(19) Europäisches Patentamt
European Patent Office
Office européen des brevets

(11) EP 1 778 703 B1

(12) # FASCICULE DE BREVET EUROPEEN

(45) Date de publication et mention de la délivrance du brevet:
**16.06.2010 Bulletin 2010/24**

(21) Numéro de dépôt: **05788449.6**

(22) Date de dépôt: **11.07.2005**

(51) Int Cl.:
***C07F 9/24*** *(2006.01)* ***C07D 207/46*** *(2006.01)*
***C07C 271/08*** *(2006.01)* ***C07F 7/18*** *(2006.01)*

(86) Numéro de dépôt international:
**PCT/FR2005/001786**

(87) Numéro de publication internationale:
**WO 2006/024722 (09.03.2006 Gazette 2006/10)**

(54) **AGENTS DE COUPLAGE A GROUPEMENT PROTECTEUR PHOTOLABILE ET LEURS UTILISATIONS, NOTAMMENT POUR LA FONCTIONNALISATION DE SUPPORTS SOLIDES**

KOPPLUNGSMITTEL MIT PHOTOLABILER SCHUTZGRUPPE UND VERWENDUNGEN DAFÜR, WIE ETWA ZUR FUNKTIONALISIERUNG VON FESTKÖRPERTRÄGERN

COUPLING AGENTS COMPRISING A PHOTOLABILE PROTECTING GROUP AND USES THEREOF, SUCH AS FOR THE FUNCTIONALISATION OF SOLID SUPPORTS

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **28.07.2004 FR 0408318**

(43) Date de publication de la demande:
**02.05.2007 Bulletin 2007/18**

(73) Titulaires:
- **Commissariat à l'énergie atomique et aux énergies alternatives 75015 Paris (FR)**
- **Université Joseph Fourier 38041 Grenoble Cédex 9 (FR)**

(72) Inventeurs:
- **HOANG, Antoine F-38000 Grenoble (FR)**
- **VINET, Françoise F-38000 Grenoble (FR)**
- **DEFRANCQ, Eric F-38830 Saint-Pierre-d'Allevard (FR)**
- **DUMY, Pascal F-38580 Allevard (FR)**

(74) Mandataire: **Goulard, Sophie et al Cabinet ORES 36 Rue de St Pétersbourg 75008 Paris Cedex (FR)**

(56) Documents cités:
**WO-A-97/39151**

- **BHUSHAN K R ET AL: "Synthesis of photolabile 2-(2-nitrophenyl)propyloxycarbonyl protected amino acids" TETRAHEDRON LETTERS, ELSEVIER SCIENCE PUBLISHERS, AMSTERDAM, NL, vol. 44, no. 47, 17 novembre 2003 (2003-11-17), pages 8585-8588, XP004464983 ISSN: 0040-4039**
- **M. ACEDO ET AL: "N-2-(2,4-dinitrophenyl) ethyloxycarbonyl-A mino Acids, New Base Labile Protected Derivatives Suitable for Solid-Phase Peptide Synthesis" TETRAHEDRON LETTERS, vol. 33, no. 34, 1992, pages 4989-4992, XP002320125**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 778 703 B1

## Description

La présente Invention est relative à des agents de couplage comportant un groupement protecteur photolabile et à leur utilisation pour la fonctionnalisation de supports solides. La présente Invention est également relative aux supports solides fonctionnalisés par ces agents de couplage, ainsi qu'à leur utilisation pour l'immobilisation de molécules biologiques d'intérêt, notamment de molécules d'acide nucléique.

Différentes méthodes permettant le greffage covalent de molécules biologiques d'intérêt, et notamment d'acide nucléique tel que les oligonucléotides (ON), sur un support solide ont été déjà été proposées. Dans le cadre particulier de la fixation d'ON, ces méthodes peuvent être divisées en deux grandes catégories :

1) La synthèse *in situ* qui consiste à construire pas à pas la molécule d'ON sur un support solide en utilisant le principe de la synthèse par la voie des phosphoramidites. Cette méthode, décrite notamment dans la demande internationale WO 97/39151 permet la préparation de surfaces à hautes densités en ON. En outre, par un jeu de caches et l'utilisation de groupes protecteurs photolabiles, elle permet l'adressage des différents ON sur le support. Néanmoins, cette méthode présente un certain nombre de désavantages majeurs. En particulier, les ON fixés sur le support ne peuvent pas être caractérisés et il est très difficile d'incorporer des ON porteurs de modifications.

2) La synthèse par déposition, ou *ex situ*, qui consiste, dans un premier temps, à préparer l'ON de manière conventionnelle, puis dans un second temps, à le fixer sur un support solide en utilisant une réaction chimique adaptée. Cette deuxième méthode présente notamment l'avantage de mettre en oeuvre des molécules d'ON qui peuvent être caractérisées avant leur fixation sur le support solide.

L'Invention qui va être décrite ci-après, s'inscrit plus spécialement dans le cadre des méthodes de synthèse par déposition.

Plusieurs types de méthodes de synthèse par déposition ont déjà été décrites dans l'art antérieur, notamment en ce qui concerne les différentes techniques permettant de localiser le greffage de l'ON.

On peut, en premier, lieu mentionner les méthodes utilisant la voie électrochimique qui consistent à ancrer l'ON, préalablement fonctionnalisé par un groupement électroconducteur tel que par exemple par un groupement pyrrole, *via* l'électropolymérisation du résidu pyrrolique. Ce type de méthode de déposition d'ON par voie électrochimique a notamment été décrit dans le brevet FR 2 703 359. Il présente néanmoins l'inconvénient de nécessiter l'emploi d'un support conducteur de l'électricité, ce qui complexifie le composant final. D'autre part, dans un composant de type "laboratoire sur puce" ("Lab On a Chip"), les différentes étapes de fabrication peuvent altérer la qualité des électrodes et donc leur fonctionnalité.

On peut, en deuxième lieu, mentionner la voie photochimique qui semble être plus prometteuse car la gestion des surfaces et des solutions chimiques à mettre en oeuvre est externalisée. Deux approches ont été développées :

i) le photogreffage direct entre la surface et la molécule cible. Dans ce cas, le photogreffage présente le désavantage de faire intervenir des réactions radicalaires peu sélectives ;

ii) la libération d'une fonction protégée par un groupement protecteur photolabile sur le support, pour permettre la réaction ultérieure avec l'ON cible pour conduire à sa fixation. Dans ce cas, les supports mis en oeuvre sont généralement fonctionnalisés par des agents de couplage qui sont des composés bifonctionnels comportant à une de leurs extrémités une fonction d'accrochage sur la surface du support et à l'autre extrémité une fonction réactive vis-à-vis d'une molécule cible comportant une fonction chimique complémentaire, ladite fonction réactive de l'agent de couplage étant protégée par un groupement photolabile. Cependant, pour être compétitive, cette dernière approche doit répondre à un certain nombre de critères. Ces critères sont les suivants :

- la réaction entre le support activé et la molécule cible doit être rapide ; cette réaction ne peut intervenir que lorsque les fonctions réactives de l'agent de couplage ont été libérées de leur groupement protecteur (réaction d'activation) ;
- le rendement de cette réaction doit être élevé ;
- le procédé de synthèse permettant de préparer les agents de couplage doit être simple à mettre en oeuvre et comporter le moins d'étapes possible ;
- l'utilisation de conditions de réactions douces (eau par exemple) ;
- la liaison formée entre l'agent de couplage et la molécule cible doit être stable dans différentes conditions de température et de pH de façon à permettre une utilisation très souple du support ainsi fonctionnalisé.

Des groupements protecteurs photolabiles de fonctions aminoacides, comme le 2-(2-nitrophényl)propyloxycarbonyl, sont utilisés pour la synthèse d'oligonucléotides sur des surfaces de verre ou d'autres types de surface via des procédés photolithographiques (Bhushan K. R. et al., Tetrahedon Letters 44, 2003, 8585-8588).

La préparation en phase solide de peptides biologiquement actifs utilise l'amino N-2-(2,4-dinitrophényl)éthyloxycarboxycarbonyl comme agent protecteur des fonctions α-amino des aminoacides (Acedo M. et al., Tetrahedon Letters, Vol. 33, No. 34, pp. 4989-4992,1992).

Or, il apparaît que les différentes méthodes proposées jusqu'à ce jour ne remplissent pas l'ensemble de ces critères de façon totalement satisfaisante, notamment en raison de la nature des différents agents de couplages utilisés.

C'est pourquoi les Inventeurs se sont donnés pour but de pourvoir à de nouveaux agents de couplages bifonctionnels comportant une fonction réactive protégée par un groupement photolabile, qui soient faciles à synthétiser, selon un procédé comportant peu d'étapes et réalisable en conditions douces, ces agents de couplage pouvant avantageusement être utilisés pour fonctionnaliser des supports solides dans le but d'y fixer ensuite des molécules biologiques cibles.

La présente Invention a donc pour premier objet un agent de couplage de formule (I) suivante :

$$A\text{-}X\text{-}B\text{-}Proc \qquad (I)$$

dans laquelle :

- A représente une fonction d'accrochage sur un support solide, ladite fonction étant choisie parmi les fonctions de type amine, phosphoramidite, silane et ester activé ;
- X représente un bras espaceur ;
- B est une fonction réactive choisie parmi les groupements conduisant, après photo-déprotection, à une fonction de type oxyamine ($-ONH_2$) et dérivés ou hydrazide ($-NH-NH_2$) et dérivés, ladite fonction B étant protégée par un groupement Proc ;
- Proc est un groupement protecteur photolabile de formule (II) suivante :

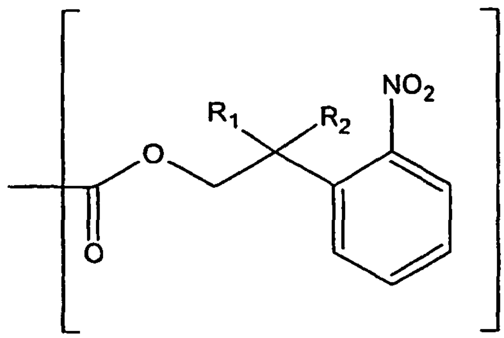

(II)

dans laquelle :

- $R_1$ et $R_2$, identiques ou différents, représentent un atome d'hydrogène ou un radical alkyle en $C_1$-$C_4$.

Selon l'Invention, la fonction A sera choisie en fonction de la nature du support sur lequel les agents de couplage de formule (I) seront destinés à être fixés. Les fonctions de type ester activé sont de préférence des fonctions acides carboxyliques estérifiées par du *N*-hydroxysuccinimide, ou encore par tout autre réactif d'activation convenable et connu de l'Homme du métier tel que par exemple le pentafluorobenzène.

Le bras espaceur X peut être de longueur et de polarité variables selon les propriétés finales souhaitées. Il peut par exemple être une chaîne hydrophobe non chargée (telle qu'une chaîne hydrocarbonée saturée) ou bien une chaîne hydrophile non chargée tel qu'un éther de glycol.

Selon une forme de réalisation particulièrement préférée de l'Invention, le bras espaceur X est choisi parmi les chaînes hydrocarbonées saturées comportant de 1 à 13 atomes de carbone et les éthers de glycol dans lesquels la chaîne carbonée comporte de 4 à 12 atomes de carbone tels que le triéthylèneglycol (TEG) et l'hexaéthylèneglycol (HEG).

La fonction réactive B est une fonction qui permet, après photo-déprotection, la fixation covalente de molécules cibles porteuses d'au moins une fonction chimique complémentaire. Ces fonctions réactives B seront aptes à interagir avec une fonction complémentaire portée par lesdites molécules cibles telle que les fonctions amines, les alcools, les thiols, les carboxyles, les carbonyles. On pourra trouver facilement, dans toute monographie de chimie organique, une liste plus exhaustive des paires de groupements fonctionnels complémentaires.

Au sein des groupements protecteurs de formule (II) ci-dessus, et lorsqu'ils représentent un radical alkyle, $R_1$ et $R_2$, indépendamment l'un de l'autre, sont de préférence choisis parmi les radicaux méthyle et éthyle ; le radical méthyle étant tout particulièrement préféré.

Parmi les agents de couplage de formule (I) ci-dessus, on préfère particulièrement ceux dans lesquels :

i) - A représente une fonction phosphoramidite,

- X représente une chaîne hydrocarbonée saturée ayant 6 atomes de carbone,
- B représente une fonction oxyamine (-ONH-), et
- $R_1$ et $R_2$ représente un atome d'hydrogène ou un radical méthyle ;

ii) - A représente une fonction trialcoxy($C_1$-$C_4$)silane,

- X représente une chaîne hydrocarbonée saturée ayant de 2 à 12 atomes de carbone,
- B représente une fonction oxyamine (-ONH-), et
- $R_1$ et $R_2$ représentent un atome d'hydrogène ou un radical méthyle ;

iii)- A représente une fonction carboxylique estérifiée par du *N*-hydroxysuccinimide ou du pentafluorobenzène,

- X représente $-CH_2-$,
- B représente une fonction oxyamine (-ONH-), et
- $R_1$ et $R_2$ représentent un atome d'hydrogène ou un radical méthyle.

Parmi les composés définis au point iii) ci-dessus, on préfère tout particulièrement les composés dans lesquels la fonction A est du triéthoxysilane et X une chaîne hydrocarbonée saturée ayant 3 ou 11 atomes de carbone.Les agents de couplages de formule (I) ci-dessus peuvent être facilement préparés selon les principes de synthèse organique bien connus de l'homme du métier et suivant la nature des fonctions A et B.

Selon une première forme de réalisation de l'Invention, et lorsque l'agent de couplage est un composé de formule (I) dans lequel la fonction d'accrochage A est de type acide carboxylique activé sous forme d'ester de *N*-hydroxysuccinimide par exemple, et la fonction B est une fonction oxyamine, on utilise de préférence un procédé de préparation répondant au Schéma 1 suivant :

**SCHEMA 1**

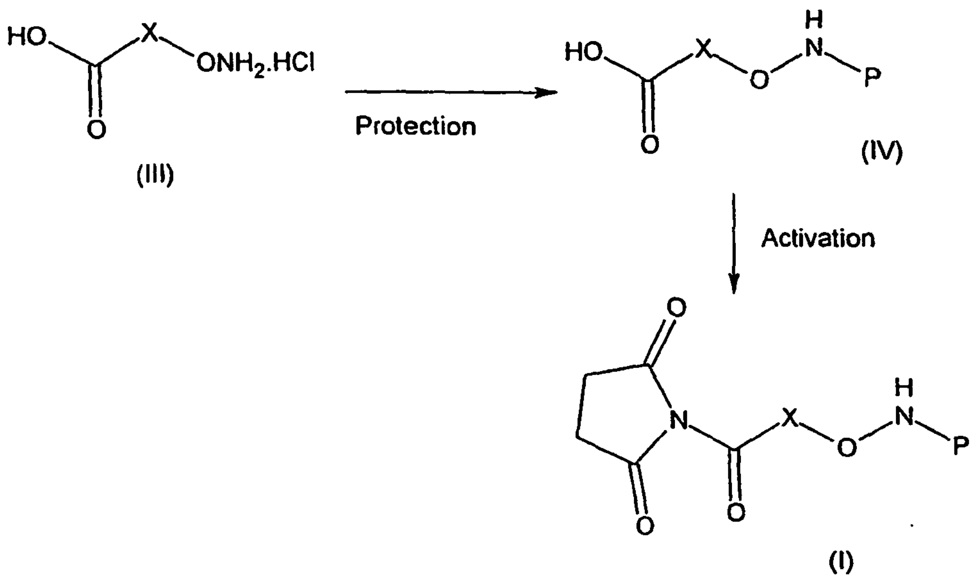


Dans ce schéma, Proc est un groupement protecteur photolabile de formule (II) telle que définie précédemment et X est un bras espaceur pouvant prendre les mêmes significations que celles indiquées précédemment.

Selon ce schéma, un composé de formule (III) comportant une fonction carboxylique et une fonction oxyamine

est, dans une première étape, protégé par un groupement protecteur photolabile Proc de formule (II) choisie, pour conduire à un composé de formule (IV), puis dans une deuxième étape, la fonction acide carboxylique du composé de formule (IV) est activée par du *N*-hydroxysuccinimide, pour conduire au composé de formule (I) attendu.

Selon une deuxième forme de réalisation de l'Invention, et lorsque l'agent de couplage est un composé de formule (I) dans lequel la fonction d'accrochage A est une fonction phosphoramidite, et la fonction B est une fonction oxyamine, on utilise de préférence un procédé de préparation répondant au Schéma 2 suivant :

**SCHEMA 2**

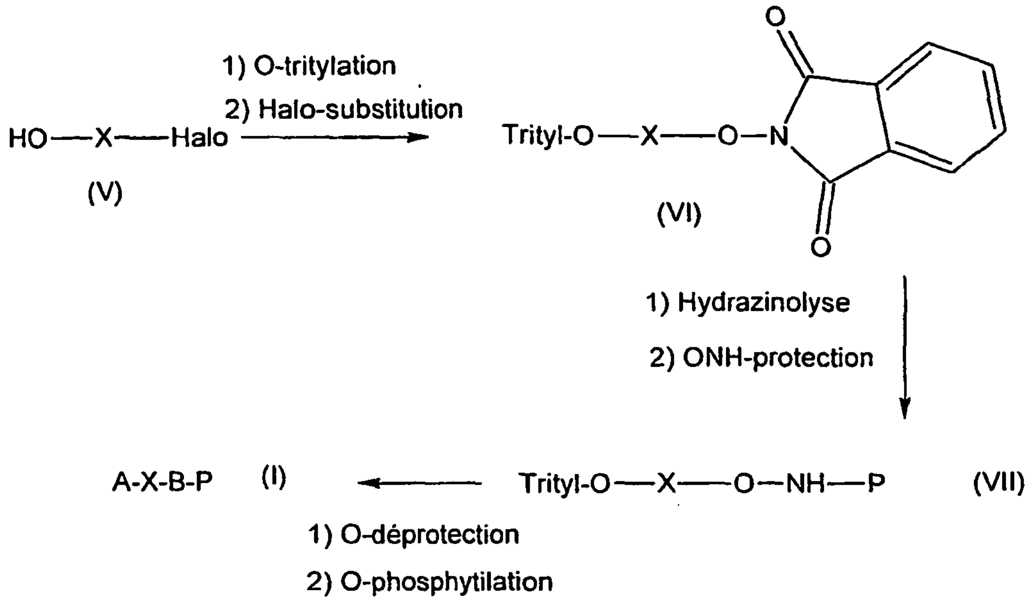


Sur ce schéma, X et Proc ont les mêmes significations que celles indiquées précédemment, B représente une fonction oxyamine et A désigne le groupement suivant :

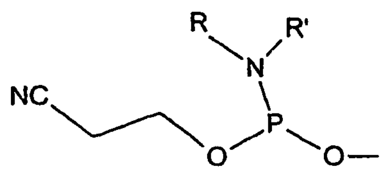

dans lequel R et R', identiques ou différents, représentent un radical alkyle ayant de 1 à 13 atomes de carbone.

Selon ce schéma, on fait réagir, dans une première étape, un alcool halogéné de formule (V) dans lequel Halo représente un atome d'halogène tel que le brome, le chlore, l'iode ou le fluor, avec du *N*-hydroxyphtalimide (substitution nucléophile de l'atome d'halogène) pour obtenir un composé de formule (VI) qui, dans une deuxième étape, subit une hydrazinolyse afin de libérer la fonction oxyamine qui est ensuite protégée par un groupement protecteur Proc photolabile de formule (II) choisie pour conduire à un composé de formule (VII) qui, dans une troisième étape, subit une phosphytilation pour conduire au composé de formule (I) attendu.

Selon une troisième forme de réalisation de l'Invention, et lorsque l'agent de couplage est un composé de formule (I) dans lequel la fonction d'accrochage A est de type silane et la fonction B est une fonction oxyamine, on utilise de préférence un procédé de préparation répondant au Schéma 3 suivant :

## SCHEMA 3

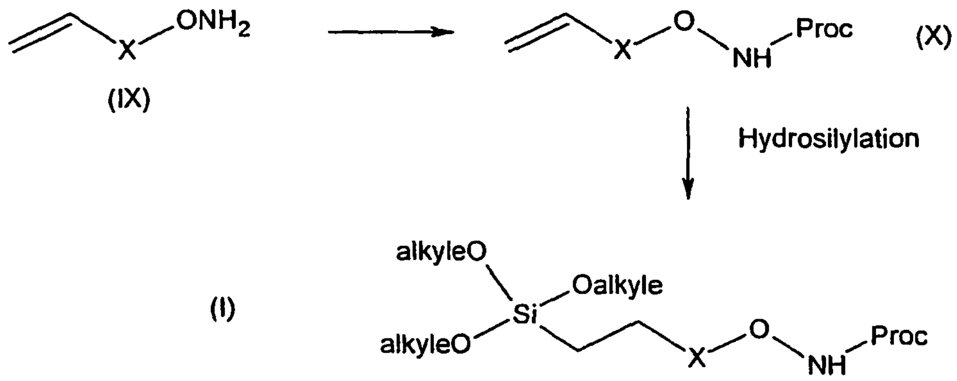


(IX) (X) (I)

Selon ce procédé, on protège la fonction oxyamine d'un composé alcène de formule (IX) dans lequel X représente un bras espaceur pouvant prendre les mêmes significations que celles indiquées précédemment pour les agents de couplage de formule (I), pour obtenir un composé de formule (X) qui subit ensuite une hydrosilylation pour conduire à un composé de formule (I) dans lequel la fonction d'accrochage A est une fonction trialcoxy($C_1$-$C_4$)silane.

Les agents de couplage de formule (I) conformes à l'Invention peuvent être utilisés pour la fonctionnalisation de supports solides. La présente Invention a donc pour objet l'utilisation d'au moins un agent de couplage de formule (I) tel que défini précédemment, pour la fonctionnalisation de supports solides.

L'utilisation des agents de couplage de formule (I) permet avantageusement de modifier rapidement la surface de supports solides par une couche stable porteuse de fonctions réactives facilement accessibles par photo-déprotection.

Ainsi, la présente Invention a également pour objet un procédé de préparation d'un support solide fonctionnalisé, caractérisé par le fait qu'il comporte au moins une étape de mise en contact d'au moins une surface d'un support solide avec une solution d'au moins un agent de couplage de formule (I) dans un solvant organique.

Le solvant organique est de préférence choisi parmi les solvants non polaires tels que par exemple le trichloroéthylène, le diméthylformamide (DMF) et le cyclohexane.

La mise en contact du support solide avec la solution de l'agent de couplage de formule (I) est de préférence réalisée à une température comprise entre 4 et 80°C environ, pendant 1 à 48 heures environ.

Le substrat est ensuite rincé avec un ou plusieurs solvants, de préférence et successivement avec le solvant de réaction, de l'éthanol absolu et/ou du chloroforme, puis séché, de préférence à l'azote.

Ce procédé présente l'avantage d'être simple à mettre en oeuvre et permet l'obtention d'une couche de bonne densité. Il permet notamment d'obtenir des supports solides comportant au moins une surface fonctionnalisée par une monocouche auto-assemblée de composés de formule (I) ("*Self-Assembled-Monolayer*" : SAM). Les SAMs sont définies comme un assemblage de molécules dans lequel les molécules sont organisées, organisation due à des interactions entre les chaînes des molécules, donnant lieu à un film anisotrope stable, monomoléculaire et ordonné (A. ULMAN, Chem. Rev., 1996, 96, 1533-1554). Il est en particulier possible d'obtenir de telles SAMs lorsque l'on fonctionnalise la surface d'un support solide avec un agent de couplage de formule (I) conforme à l'Invention dans lequel la fonction d'accrochage A est une fonction de type silane, de préférence en solution à 10 mM environ dans un solvant tel que le trichloroéthylène et lorsque la mise en contact est réalisée à une température d'environ 4°C pendant environ 24 heures.

Par ailleurs, les surfaces obtenues en mettant en oeuvre le procédé conforme à l'Invention présentent directement un grand nombre de fonctions réactives protégées par un groupement protecteur photolabile facile à enlever de façon sélective dans le temps et l'espace afin d'immobiliser, de façon covalente, des molécules biologiques d'intérêt comportant une fonction chimique complémentaire vis-à-vis de la fonction réactive B des agents de couplages présents à la surface du support solide.

Les supports solides pouvant être fonctionnalisés par les agents de couplage de formule (I) conformes à l'Invention sont de préférence choisis parmi les supports de verre, de céramiques (de type oxyde), de silicium ou de plastique, lesdits supports comportant au moins une surface hydratée, hydroxylée, silanisée, aminée ou bien encore de type ester activé. De telles surfaces peuvent être aisément préparées selon les techniques bien connues de l'art antérieur de la technique. Il est par exemple possible de préparer un support solide comportant une surface hydroxylée par silanisation au moyen d'un silane époxyde qui subit ensuite une hydrolyse acide de façon à libérer les fonctions hydroxyle de la surface.

Ces supports solides possèdent au moins une surface plane ou non, lisse ou structurée, et peuvent se présenter

par exemple sous la forme de lame de verre, de plaque plastique plane ou à puits, de capillaire ou encore de bille poreuse ou non.

Ainsi que décrit ci-dessus, la fonction d'accrochage A des agents de couplage de formule (I) est choisie en fonction de la nature de la surface du support sur laquelle ceux-ci sont destinés à être fixés.

Ainsi lorsqu'il s'agit de supports comportant au moins une surface de type ester activé, alors la fonction A est de préférence une fonction amine ; lorsqu'il s'agit de supports comportant au moins une surface de type hydroxyle, alors la fonction A est de préférence une fonction phosphoramidite ou silane ; lorsqu'il s'agit de supports comportant au moins une surface de type hydrure, alors la fonction A est de préférence une fonction silane et lorsqu'il s'agit de supports comportant une surface aminé, alors la fonction A est de préférence une fonction ester activée.

La présente Invention a donc également pour objet les supports solides comportant au moins une surface fonctionnalisée par un ou plusieurs agents de couplage de formule (I) tels que définis ci-dessus.

De tels supports peuvent avantageusement être utilisés pour l'immobilisation de molécules biologique d'intérêt comportant une fonction chimique complémentaire vis-à-vis de la fonction réactive B des agents de couplage de formule (I) présents à la surface dudit support solide après leur déprotection, et en particulier pour l'immobilisation covalente de molécules d'acide nucléique telles que l'ADN et les oligonucléotides.

Ainsi, la présente Invention a également pour objet l'utilisation d'un support solide tel que décrit ci-avant pour l'immobilisation covalente de molécules biologiques d'intérêt comportant une fonction chimique complémentaire vis-à-vis de la fonction réactive B des agents de couplage de formule (I) présents à la surface dudit support solide, et en particulier d'acides nucléiques (ADN, oligonucléotides) par formation d'une liaison amide (peptidique).

La présente Invention a, en outre, pour objet un procédé d'immobilisation de molécules biologiques d'intérêt, et en particulier de molécules d'acide nucléique, sur un support solide tel que décrit ci-avant, **caractérisé en ce qu**'il comprend au moins une première étape de photo-déprotection des fonctions réactives B composés de formule (I) par insolation d'au moins une partie de la surface du support solide, puis la mise en contact du support solide ainsi activé avec une solution de molécules biologiques d'intérêt, et en particulier de molécules d'acide nucléique, pour conduire à l'immobilisation desdites molécules par formation d'une liaison covalente entre au moins une fonction chimique portée par lesdites molécules et réactive vis-à-vis des fonctions réactives B desdits composés de formule (I) conforme à l'Invention, sur ledit support solide et la répétition éventuelle de ces deux étapes.

En particulier, l'étape de photo-déprotection peut-être localisée sur seulement une partie de la surface fonctionnalisée du support solide et par exemple réalisée au travers d'un masque.

Après insolation, la fonction réactive B des agents de couplage de formule (I) se trouve déprotégée (active), ce qui crée ainsi des zones portant la fonction réactive libre qui pourra capturer toute molécule en solution comportant une fonction chimique réactive vis-à-vis de ladite fonction réactive B activée présente à la surface du support solide.

En particulier, l'étape d'immobilisation est réalisée par la formation de liaisons oxime entre les fonctions B oxyamine des composés de formule (I) et les fonctions carbonylées des molécules biologiques d'intérêt, en particulier des molécules d'acide nucléique. Cette réaction présente l'avantage de pouvoir être réalisée dans des conditions douces, en particulier à un pH compris entre 4 et 7. La liaison formée (oxime) présente par ailleurs une grande stabilité et il n'est pas nécessaire de la stabiliser par une étape de réduction supplémentaire.

La longueur d'onde d'insolation est de préférence comprise entre 300 et 400 nm.

Les solutions de molécules biologiques d'intérêt peuvent être de simples solutions aqueuses ou bien des solutions de ces molécules dans des solutions aqueuses tamponnées.

La durée de la mise en contact est de préférence comprise entre 5 et 60 minutes environ et réalisée à une température de préférence comprise entre 4 et 60°C environ.

Enfin, l'Invention a également pour objet les supports solides (puces à acides nucléiques notamment) tels que décrits précédemment et obtenus en mettant en oeuvre le procédé d'immobilisation conforme à l'Invention, c'est-à-dire comportant au moins une surface sur laquelle lesdites molécules biologiques d'intérêt, et en particulier des molécules d'acide nucléique, sont immobilisées par l'intermédiaire d'une liaison covalente formée avec la fonction réactive B des agents de couplage de formule (I) conformes à l'Invention.

Outre les dispositions qui précèdent, l'Invention comprend encore d'autres dispositions qui ressortiront de la description qui va suivre, qui se réfère à des exemples de préparation d'agents de couplage de formule (I), à des exemples de fonctionnalisation de supports solides en verre de type capillaire ou support solide plan avec des agents de couplages de formule (I) conforme à l'Invention, à l'utilisation de ces supports fonctionnalisés pour l'immobilisation d'oligonucléotides, ainsi qu'aux figures 1 et 2 annexées dans lesquelles :

- la figure 1 représente la photographie de la fluorescence émise par des oligonucléotides fixés sur un support solide de type capillaire selon le procédé conforme à l'Invention, après hybridation avec des oligonucléotides complémentaires marqués à l'aide d'un fluorophore (capillaire de type a/), comparativement à un capillaire non fonctionnalisé (capillaire de type b/) ;
- la figure 2 représente la photographie de la fluorescence émise par des oligonucléotides fixés sur un support solide

plan selon le procédé conforme à l'Invention, après hybridation avec des oligonucléotides complémentaires marqués à l'aide d'un fluorophore (support de type a/), comparativement à un support non fonctionnalisé (support de type b/).

## EXEMPLE 1 : PREPARATION D'UN DERIVE PHOSPHORAMIDITE CONFORME A LA FORMULE (I)

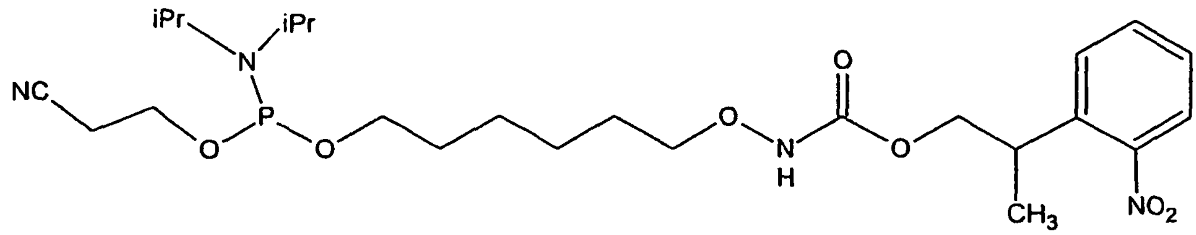

(6)

1) Première étape : Préparation du composé **(1)** : introduction de l'oxyamine

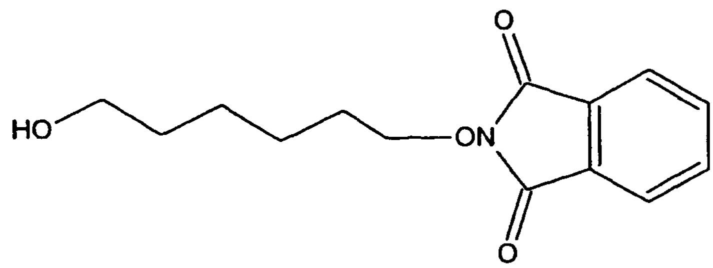

(1)

Une solution de *N*-hydroxyphtalimide (2,70 g ; 16,6 mmoles) et de carbonate de potassium (4,6 g ; 23,6 mmoles) dans 150 ml de diméthylformamide (DMF) a été chauffée à une température de 50°C sous argon pendant une heure sous agitation. On a ensuite ajouté du 6-bromohexanol (3 g ; 16,6 mmoles), puis le mélange réactionnel a de nouveau été mis sous agitation pendant une nuit à une température de 50°C. Après filtration, le solvant a été évaporé sous pression réduite. On a ensuite ajouté au résidu ainsi obtenu 50 ml d'acétate d'éthyle puis la phase organique a été lavée avec de la soude 0,1 N, puis avec une solution aqueuse saturée en chlorure de sodium. La phase organique a ensuite été séchée sur sulfate de sodium anhydre puis évaporée. Le produit **(1)** a été obtenu sous la forme d'une poudre blanche (3,28 g ; 12,5 mmoles ; rendement 75 %) présentant un point de fusion compris entre 85 et 88°C.

L'analyse en résonance magnétique nucléaire du proton **(RMN -$^1$H)**, effectuée à une longueur d'onde de 200 MHz dans du CDCL3 était la suivante : δ ppm = 1,30-1,90 (8H, m, 4 $C\underline{H}_2$), 3,60 (2H, t, J = 13 Hz, $C\underline{H}_2O$), 4,20 (2H, t, J = 13 Hz, $C\underline{H}_2ON$), 7,70-7,90 (4H, m, Ar-H).

**SM** (DCI, mode positif) : $M_{calc}$ - 263 ($C_{14}H_{17}NO_4$), m/z = 281 $[M+NH_4]^+$.

2) Deuxième étape, : Tritylation du composé **(1)** pour conduire au composé **(2)**

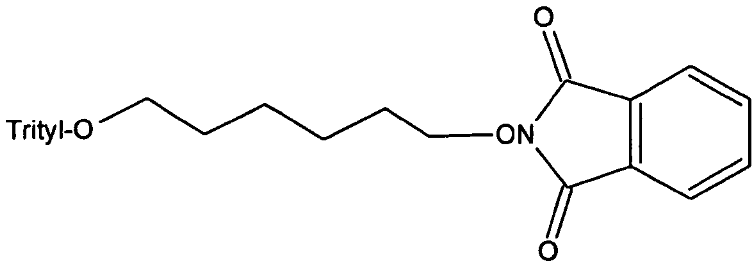

(2)

A une solution du composé **(1)** obtenu ci-dessus à l'étape précédente (3,2 g ; 12,5 mmoles) dans 75 ml de pyridine anhydre, on a ajouté du chlorure de trityle (4,2 g ; 15 mmoles). La solution a été agitée sous argon pendant 1 nuit à température ambiante. 5 ml de méthanol ont ensuite été ajoutés lentement, puis le solvant a été évaporé sous pression réduite. Le résidu obtenu a alors été solubilisé dans 100 ml d'acétate d'éthyle, puis la phase organique a été lavée avec de l'eau puis avec une solution aqueuse saturée en chlorure de sodium. La phase organique a alors été séchée sur sulfate de sodium anhydre puis évaporée. Le produit brut a été purifiée par chromatographie sur gel de silice (éluant dichlorométhane/cyclohexane : 75/25, v/v). Le produit **(2)** a été obtenu sous la forme d'une poudre blanche (6,27 g, 12,4 mmoles ; rendement 99 %) fondant à 106-107°C.

L'analyse **RMN-$^1$H** (200MHz, $CDCL_3$) était la suivante : δ ppm = 1,30-1,90 (8H, m, 4 $C\underline{H}_2$), 3,20 (2H, t, J = 13 Hz, $C\underline{H}_2O$), 4,20 (2H, t, J = 13 Hz, $C\underline{H}_2ON$), 7,70-7,90 ($4H_{Ar}$, m, Ar-$\underline{H}$-phtalimide), 7,20-7,40 (15H, m, Ar-$\underline{H}$-trityle).

**SM** (ESI, mode positif) : $M_{Calc}$ = 505 ($C_{33}H_{31}NO_4$), m/z = 528 $[M+Na]^+$, m/z = 544 $[M+K]^+$.

3) Troisième étape : Hydrazinolyse du composé **(2)** pour l'obtention du composé **(3)**

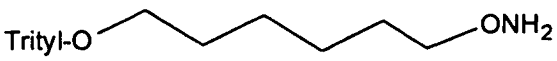

(3)

Le composé **(2)** obtenu ci-dessus à la deuxième étape (6,27 g ; 12,4 mmoles) a été solubilisé dans 50 ml de dichlorométhane et l'on a ajouté 780 mg d'hydrazine (24,8 mmoles). La solution obtenue a été portée au reflux pendant 1 heure et demie, puis filtrée et évaporée sous pression réduite. Le produit brut a été purifié par chromatographie sur gel de silice, avec comme éluant du dichlorométhane puis un mélange 95/5 (v/v) de dichlorométhane et de méthanol. On a obtenu le composé (3) sous la forme d'une huile (4,20 g ; 11,2 mmoles) avec un rendement de 90 %.

**RMN-$^1$H** (200 MHz, $CDCl_3$) δ ppm = 1,30-1,80 (8H, m, 4 $C\underline{H}_2$), 3,10 (2H, t, J = 13 Hz, $C\underline{H}_2$-Tri), 3,60 (2H, t, J = 13 Hz, $C\underline{H}_2ON$), 7,70-7,90 (15H, m, Ar-$\underline{H}$-trityle).

**SM** (ESI, mode positif) : $M_{calc}$ = 375 ($C_{25}H_{29}NO_2$), m/z = 376 $[M+H]^+$, m/z = 398 $[M+Na]^+$, m/z = 414 $[M+K]^+$.

4) Quatrième étape : Protection de l'oxyamine du composé **(3)** pour obtenir le composé **(4)**

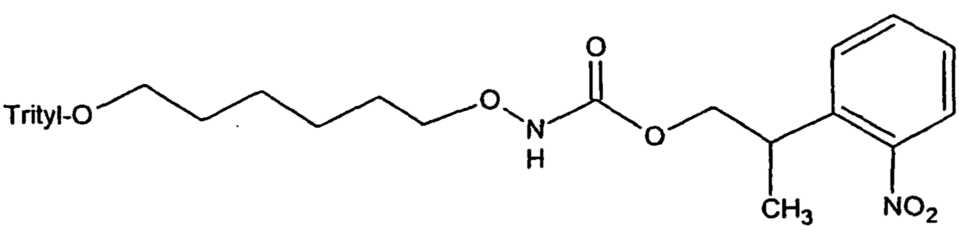

(4)

Le composé **(3)** préparé ci-dessus à la troisième étape (4,20 g ; 11,2 mmoles) a été dissous dans 100 ml de pyridine. On a ensuite ajouté, goutte à goutte, 50 ml d'une solution de dichlorométhane contenant 5,40 g (22,4 mmoles) de chloroformiate de 2-(2-nitrophényl)-propyle (Cl-NPPOC). Le mélange réactionnel a été agité pendant une heure à

l'obscurité puis évaporé sous pression réduite. Le résidu obtenu a ensuite été solubilisé dans du dichlorométhane puis la phase organique a été lavée avec une solution aqueuse saturée en chlorure de sodium. La phase organique a été séchée sur sulfate de sodium anhydre puis évaporée. Le produit brut a été purifié par chromatographie sur gel de silice en utilisant le dichlorométhane comme éluant. Le produit **(4)** a été obtenu sous la forme d'une huile orange (5,80 g ; 9,9 mmoles) avec un rendement de 89 %.

RMN-$^1H$ (200 MHz, $CDCl_3$) : δ ppm = 1,30 (3H, d, J = 16 Hz, C<u>H</u>$_3$), 1,50-1,80 (8H, m, 4 C<u>H</u>$_2$), 3,0 (2H, t, J = 13 Hz, C<u>H</u>$_2$O-Tri), 3,70 (2H, m, C<u>H</u>$_2$ON, 1H, m, C<u>H</u>), 4,20 (2H, t, J = 13 Hz, C<u>H</u>$_2$OCO), 7,10-7,50 (15H, m, Ar-<u>H</u>-trityle), 7,50-7,80 (4H, m, Ar-<u>H</u> NPPOC).

**SM** (ESI, mode positif) : $M_{calc}$ = 582 ($C_{35}H_{38}N_2O_6$), m/z = 605 $[M+Na]^+$, m/z = 621 $[M+K]^+$.

**SM** (ESI, mode négatif) : m/z = 581 $[M-H]^-$.

<u>5) Détritylation du composé **(4)** pour obtenir le composé **(5)**</u>

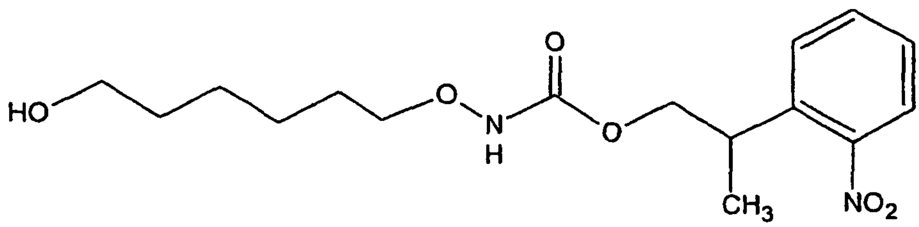

**(5)**

5,80 g (9,90 mmoles) du composé **(4)** obtenu ci-dessus à l'étape précédente ont été dissous dans 50 ml d'une solution d'acide trifluoroacétique à 20 % dans le dichlorométhane. Le mélange a été agité pendant 3 à 4 heures à température ambiante, puis évaporé sous pression réduite. Le résidu obtenu a été solubilisé dans 100 ml de dichlorométhane, puis la phase organique a été lavée avec une solution aqueuse saturée en NaCl. La phase organique a alors été séchée sur sulfate de sodium anhydre puis évaporée. Le produit brut a été purifié par chromatographie sur gel de silice en utilisant comme éluant du dichlorométhane puis un mélange dichlorométhane/méthanol 95/5 (v/v). Le composé **(5)** a été obtenu sous la forme d'une huile orange (3,0 g ; 8,9 mmoles) avec un rendement de 90 %.

**RMN-$^1$H** (200 MHz, $CDCl_3$) : δ ppm = 1,30 (3H, d, J = 16 Hz, C<u>H</u>$_3$), 1,50-1,80 (8H, m, 4 $CH_2$), 3,70 (2H, m, C<u>H</u>$_2$ON, 1H, m, C<u>H</u>), 4,30 (2H, t, C<u>H</u>$_2$OCO, 2H, t, C<u>H</u>$_2$OH), 7,50-7,80 (4H, m, Ar-H NPPOC).

**SM** (DVI, mode positif) : $M_{calc}$ = 340 ($C_{16}H_{24}N_2O_6$), m/z = 454 $[M+TFA]^+$.

<u>6) Sixième étape : Phosphitylation du composé **(5)** pour conduire au composé **(6)**</u>

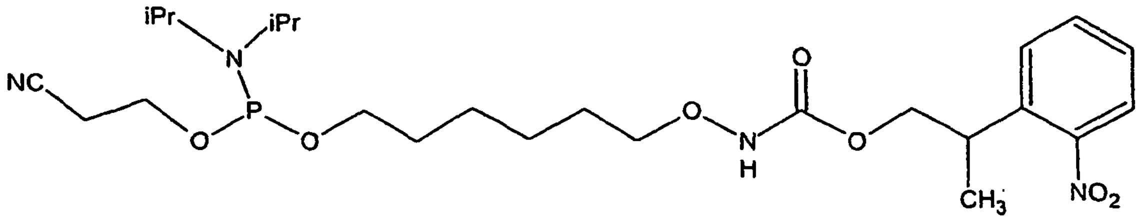

**(6)**

Le composé **(5)** obtenu ci-dessus à l'étape précédente (0,90 g ; 2,7 mmoles) a été solubilisé sous courant d'argon dans 15 ml de dichlorométhane, puis on a ajouté de la diisopropyléthylamine (DIEA) (0,56 ml, 3,2 mmoles), puis 0,72 ml de 2-cyanoéthyldiisopropylchlorophosphoramidite (3,2 mmoles). Le mélange réactionnel a été agité à température ambiante pendant 4 heures, jusqu'à disparition du produit de départ. On a alors ajouté 40 ml de dichlorométhane, puis lavé la phase organique avec une solution d'hydrogénocarbonate de sodium saturée, puis avec une

solution aqueuse saturée en NaCl. La phase organique a été séchée sur sulfate de sodium anhydre puis évaporée. Le produit brut a été purifié par chromatographie sur gel de silice en utilisant comme éluant du dichlorométhane puis un mélange dichlorométhane/méthanol 97/3 (v/v). Le composé **(6)** a été obtenu sous la forme d'une huile jaune (0,50 g ; 1,0 mmoles) avec un rendement de 38 %.

**RMN-$^1$H** (200 MHz, $CDCl_3$) : $\delta$ ppm = 1,20-1,50 (15H, m, 4 $CH_3$), 1,50-1,85 (8H, m, 4 $CH_2$), 2,65 (2H, t, $CH_2CN$) 3,80 (2H, m, $CH_2OH$, 1H, m, CH, 4 H, m, 2 $CH_2OP$), 4,30 (2H, m, $CH_2OCO$, 2H, m, 2 CHN), 7,10-7,70 (4H Ar-H NPPOC).

**RMN- $^{31}$P** (200 MHz, $CDCl_3$) : $\delta$ ppm = 120 (2 diastéréo-isomères).

**EXEMPLE 2 : PREPARATION D'UN DERIVE ESTER ACTIVE CONFORME A LA FORMULE (I) (composé (8))**

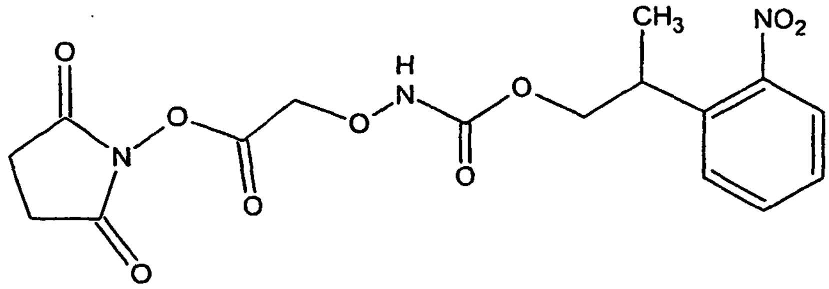

**(8)**

1) Première étape : Préparation de l'acide précurseur **(7)**

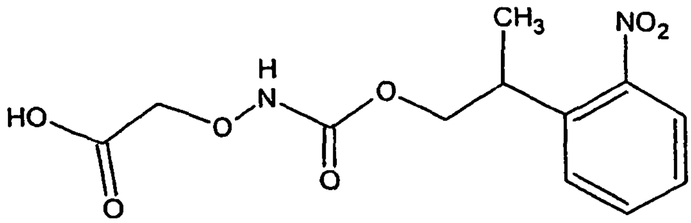

**(7)**

Du chlorhydrate de carboxyméthoxylamine (1 g ; 4,57 mmoles) a été dissous dans 25 ml d'une solution aqueuse de carbonate de sodium à 10 %. La solution a été refroidie à une température de 0°C et l'on a ajouté, goutte à goutte et sous agitation, 20 ml d'une solution de dioxane renfermant 2,20 g (9,1 mmoles) de chloroformiate de 2-(2-nitrophényl)-propyle (Cl-NPPOC). L'agitation a été maintenue pendant 2 à 3 heures à température ambiante. Le milieu réactionnel a été évaporé à sec. Au résidu ainsi obtenu, on a ajouté 250 ml d'eau, puis on a lavé la phase aqueuse avec 200 ml d'éther diéthylique. La phase aqueuse a alors été acidifiée avec une solution d'acide chlorhydrique 1N jusqu'à pH 3 et l'on a extrait avec trois fois 250 ml de dichlorométhane. Les phases organiques ont été réunies et séchées sur sulfate de sodium anhydre. Le produit brut a été purifié par chromatographie sur gel de silice en utilisant comme éluant du dichlorométhane puis un mélange dichlorométhane/méthanol 97/3 (v/v). Le composé **(7)** a été obtenu sous la forme d'une poudre blanche fondant entre 88 et 92°C (1,20 g ; 4,0 mmoles), avec un rendement de 90 %.

**RMN-$^1$H** (200 MHz, $CDCl_3$) : $\delta$ ppm = 1,40 (3H, d, J = 16 Hz, $CH_3$), 3,70 (1H, m, CH), 4,30 (2H, m, $CH_2O$) 4,40 (s, 2H, $COCH_2O$), 7,30-7,60 (4H, m, Ar-H NPPOC).

**RMN-$^{13}$C** (300 MHz, $CDCl_3$) : $\delta$ ppm = 18 ($CH_2$), 33 ($CH_2$), 67 ($CH_3$), 70 ($CH_3$), 75 ($CH_3$), 123 (quat), 137 (quat), 138 (quat), 133 (quat), 136 (ÇH), 151 (CH), 159 (CH), 172 (CH), 173 (CH).

**SM** (ESI, mode positif) : $M_{calc}$ = 298 ($C_{12}H_{14}N_2O_7$), m/z = 321 $[M+Na]^+$, m/z = 337 $[M+K]^+$.

SM (ESI, mode négatif) : m/z = 297 $[M-H]^-$, m/z = 595 $[2M-H]^-$.

2) Deuxième étape : préparation de l'ester activé N-hydroxysuccinimide (8)

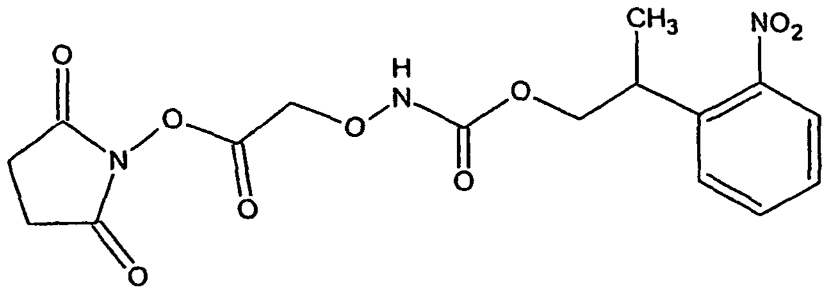

(8)

L'acide **(7)** obtenu ci-dessus à l'étape précédente (1,20 g ; 4,0 mmoles) a été dissous dans 15 ml de dichlorométhane anhydre, puis l'on a ajouté du dicyclocarbodiimide (DCC) (0,82 g ; 4,4 mmoles) puis du *N*-hydroxysuccinimide (0,457 g ; 4,4 mmoles). Le milieu réactionnel a été agité pendant une nuit. Après filtration, le solvant a été évaporé sous pression réduite. Le produit brut a été purifié par chromatographie rapide sur gel de silice en utilisant de l'acétate d'éthyle comme éluant. Le composé **(8)** a été obtenu sous la forme d'une poudre jaune (1,0 g ; 2,68 mmoles) avec un rendement de 67 %.

**EXEMPLE 3 : PREPARATION D'UN DERIVE ESTER DE FORMULE (I), ACTIVE AVEC DU PENTAFLUOROPHENOL (9)**

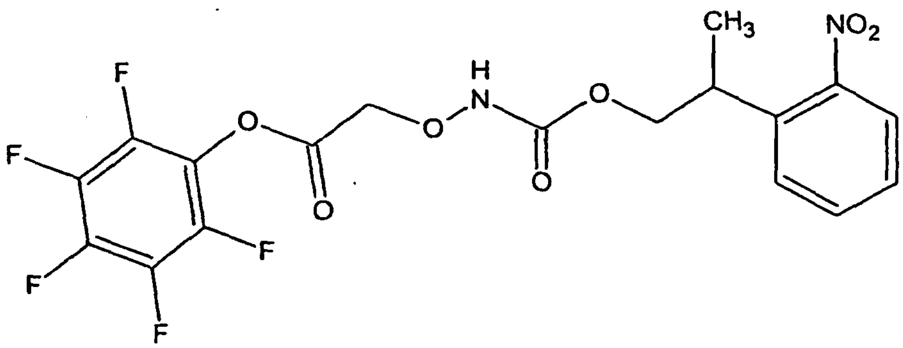

(9)

500 mg (1,6 mmoles) de composé **(7)** obtenu à la première étape de l'exemple 2 ci-dessus ont été dissous dans 5 ml de dichlorométhane, puis l'on a ajouté 372 mg (1,9 mmoles) de pentafluorophénol, puis goutte à goutte, 1 ml d'une solution de DCC (360 mg, 1,9 mmoles) dans le dichlorométhane. On a agité pendant 4 heures, puis le précipité de dicyclourée (DCU) formé a été filtré. Après évaporation du solvant, on a obtenu le composé **(9)** sous la forme d'une huile (740 mg, 1,9 mmoles), avec un rendement de 100 %.

**RMN-$^{1}$H** (200 MHz, $CDCl_3$) : δ ppm = 1,30 (3H, d, J = 16 Hz, $C\underline{H}_3$), 3,70 (1H, m, $C\underline{H}$), 4,30 (2 H, m, $C\underline{H}_2O$) 4,70 (s, 2H, $COC\underline{H}_2O$), 7,30-7,70 (4H, m, Ar-$\underline{H}$ NPPOC).

**RMN-$^{19}$F** (200 MHz, $CDCl_3$) : δ ppm = -166,0 (1F, t), -164,0 (1F, d), -162,0 (1F, t), -157,0 (1F,t), -152,50 (1F, d).

**EXEMPLE 4 : PREPARATION D'UN DERIVE TRIETHOXYSILANE DE FORMULE (I)**

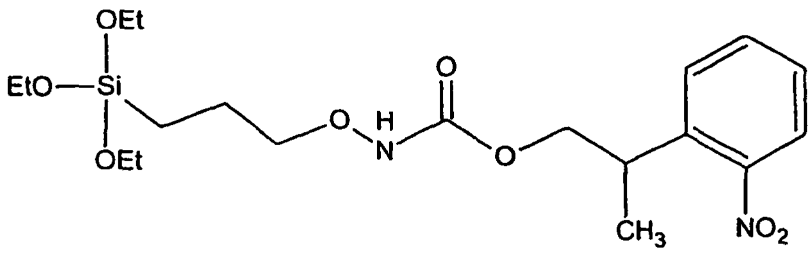

(11)

1) Première étape : Préparation de la *N*-nitro-phényl-propyloxycarbonyl-allyloxyamine **(10)**

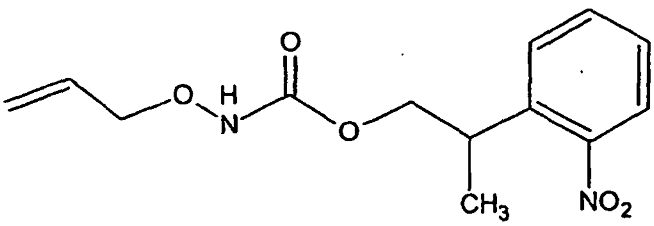

(10)

A une solution de 3,250 g (29,6 mmoles ; 1,1 équivalent) d'allyloxyamine commerciale dans 25 ml de pyridine anhydre refroidie à une température de 0°C, on a ajouté, goutte à goutte, 15 ml d'une solution de dichlorométhane renfermant 6 g (27 mmoles) de chloroformiate de 2-(2-nitrophényl)-propyle (Cl-NPPOC). Après 30 minutes d'agitation, le solvant a été évaporé puis co-évaporé avec du toluène. Le produit brut réactionnel a été dissous dans de l'acétate d'éthyle. La phase organique a été lavée, séchée sur sulfate de sodium anhydre, puis évaporée à pression réduite. Le composé **(10)** a été obtenu après purification par chromatographie sur gel de silice en utilisant comme éluant un mélange acétate d'éthyle/hexane : 1/4 (v/v) (5,76 g), avec un rendement de 72 %.
**RMN-$^1$H** (200 MHz, $CDCl_3$) : δ ppm = 1,40 (d, 6H, Si-$CH_2$-$\underline{H}_3$), 3,70 (q, 1H, Ar-CH-$CH_3$), 4,30 (m, 4H, $C\underline{H}_2$O, $OC\underline{H}_2$) 5,30 (dd, 2H, $C\underline{H}_2$), 5,90 (m, 1H, C$\underline{H}$), 7,20 (s, 1H, NH), 7,30-7,80 (m, 4H, $\underline{H}$-Ar).

2) Deuxième étape : Hydrosilylation pour conduire au composé **(11)**

A une solution de 1,67 g (5,6 mmoles) du composé (10) obtenu ci-dessus à l'étape précédente dans 8 ml de triéthoxysilane, on a ajouté goutte à goutte 20 µl de catalyseur de Karsted. Après une nuit d'agitation à une température de 60°C, le solvant a été évaporé par distillation sous vide. Le produit brut réactionnel a été purifié par chromatographie sur gel de silice en utilisant comme éluant un mélange acétate d'éthyle/hexane 1/4 (v/v) pour conduire au composé attendu **(11)** avec un rendement de 60 % (1,33 g).
**RMN-$^1$H** (200 MHz, $CDCl_3$) : δ ppm = 0,65 (m, 2H, $C\underline{H}_2$-Si), 1,20 (t, 3H, $C\underline{H}_3$-$CH_2$-O), 1,40 (d, 3H, $C\underline{H}_3$-CH) 1,70 (m, 3H, $C\underline{H}_2$, C$\underline{H}$-$CH_3$), 3,80 (m, 4H, O-$C\underline{H}_2$-$CH_3$, $C\underline{H}_2$-O), 4,30 (m, 2H, $C\underline{H}_2$-O-CO), 7,30-7,80 (m, 5H, N$\underline{H}$, $\underline{H}$-Ar).
SM (électrospray, mode positif) : $M_{calc}$ = 444, m/e = 444,7 $[M+H]^+$.
Les exemples de préparation de supports solides qui suivent ont été réalisés avec les agents de couplage de formule (I) conformes à l'Invention, en format capillaire (capillaires de verre d'une longueur de 3 cm et d'un diamètre de 100 µm). Il doit être bien entendu toutefois que ces exemples sont transposables en format plan sur d'autres types de support.

## EXEMPLE 5 : PREPARATION D'UN SUPPORT SOLIDE FONCTIONNALISE AVEC UN AGENT DE COUPLAGE DE FORMULE (I) COMPORTANT UNE FONCTION D'ACCROCHAGE A DE TYPE SILANE

Dans cet exemple le composé (11) tel que préparé ci-dessus à l'exemple 4 a été utilisé à titre d'agent de couplage de formule (I).
L'obtention d'un support solide fonctionnalisé par le composé **(11)** se fait en une seule étape, en l'occurrence par l'étape dite de silanisation de la surface du support solide minéral (capillaire de verre) selon le schéma réactionnel ci-dessous :

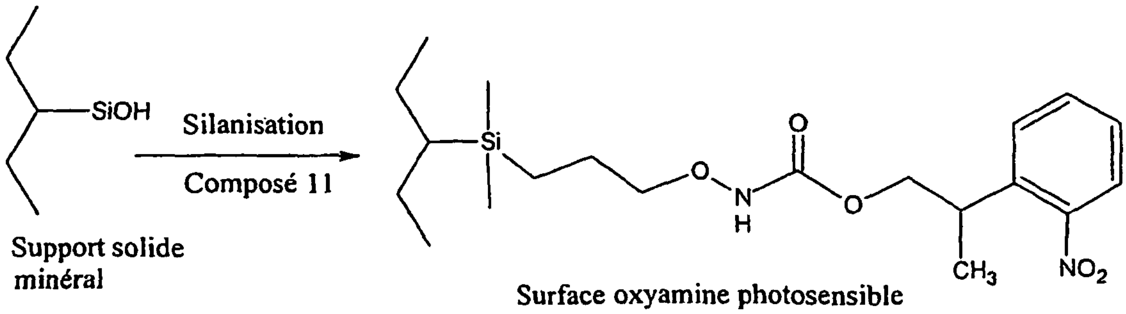


Pour ce faire, un capillaire a été rempli avec une solution de soude à 6M dans un mélange 1/1 eau/éthanol et la solution a été laissée dans le capillaire durant 2 heures à température ambiante.

On a ensuite rincé abondamment le capillaire avec de l'eau puis on l'a séché à l'azote.

Le capillaire a ensuite été rempli avec une solution du composé **(11)** à 10 mM dans du trichloroéthylène et on a laissé agir durant une nuit à température ambiante.

Le capillaire a ensuite été rincé abondamment avec du trichloroéthylène, de l'éthanol absolu puis du chloroforme et enfin séché à l'azote.

On a ainsi obtenu un capillaire de verre dont la surface interne était fonctionnalisée par une couche de composé **(11),** c'est-à-dire une surface oxyamine photosensible.

**EXEMPLE 6 : PREPARATION D'UN SUPPORT SOLIDE FONCTIONNALISE PAR UN AGENT DE COUPLAGE DE FORMULE (I) COMPORTANT UNE FONCTION D'ACCROCHAGE A DE TYPE ESTER ACTIVE**

Dans cet exemple le composé **(8)** tel que préparé ci-dessus à l'exemple 2, comportant une fonction d'accrochage A de type ester activé par NHS a été utilisé à titre d'agent de couplage de formule (I).

L'obtention d'un support solide fonctionnalisé par cet agent de couplage ester activé NHS se fait en deux étapes, selon le schéma réactionnel ci-dessous :

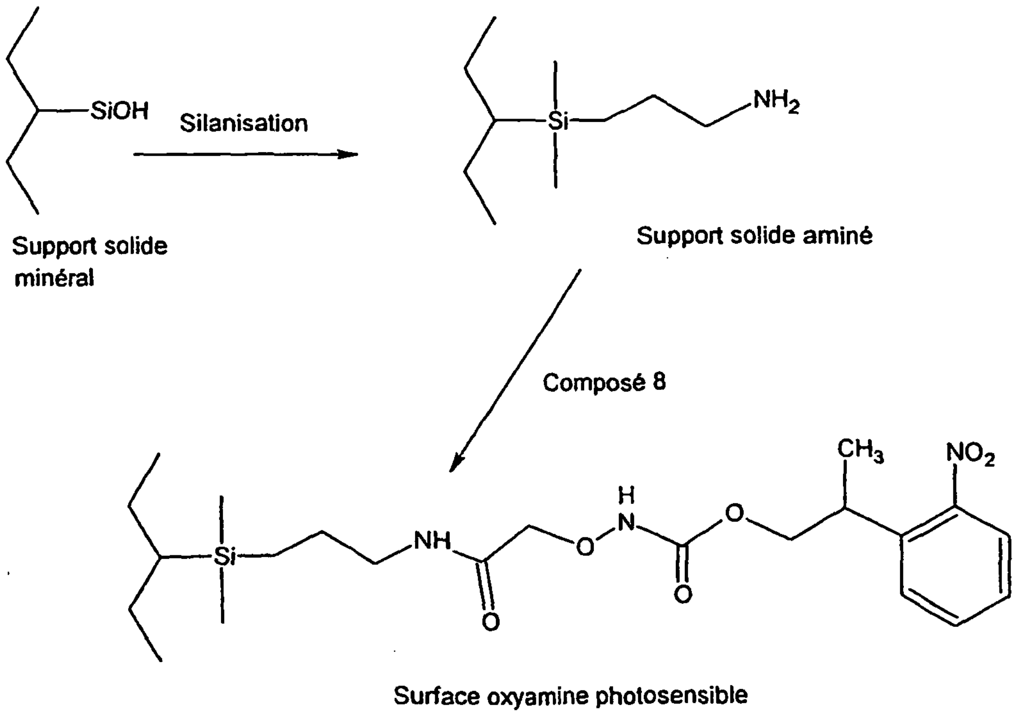


selon lequel, dans une première étape, on transforme la surface minérale du support solide en une surface comportant des fonctions amine, et dans une deuxième étape on fait réagir lesdites fonctions amine de surface avec le composé **(8).**

1) Première étape : réalisation d'une surface comportant des fonctions amine

Un capillaire de verre a été rempli avec une solution de soude à 6M dans un mélange 1/1 eau/éthanol et on a laissé la solution dans le capillaire durant 2 heures à température ambiante.
On a rincé abondamment le capillaire avec de l'eau puis on l'a séché à l'azote.
Le capillaire a ensuite été rempli avec une solution de triéthoxy-aminopropyl-silane à 10 % dans l'éthanol à 95° et on a laissé la solution dans le capillaire durant une nuit à température ambiante. Le capillaire a ensuite été rincé avec de l'éthanol puis séché à l'azote.
On a obtenu un capillaire dont la surface interne était recouverte de fonctions amine.

2) Deuxième étape : fonctionnalisation du support avec le composé **(8)**

Le capillaire obtenu ci-dessus à la première étape a été rempli d'une solution du composé **(8)** à 10 mM dans le diméthylformamide (DMF) et on a laissé la solution dans le capillaire durant 1 nuit à température ambiante.
Le capillaire a ensuite été rincé avec du DMF puis de l'éthanol et enfin séché à l'azote.
On a ainsi obtenu un capillaire de verre dont la surface interne était fonctionnalisée par une couche de composé **(8),** c'est-à-dire une surface oxyamine photosensible.

**EXEMPLE 7 : UTILISATION D'UN SUPPORT FONCTIONNALISE POUR L'IMMOBILISATION DE MOLECULES BIOLOGIQUES D'INTERET**

Dans cet exemple, le support solide obtenu ci-dessus à l'exemple 5 a été utilisé pour l'immobilisation d'oligonucléotides (ON). Il doit être bien entendu toutefois que l'utilisation de ces supports pour l'immobilisation d'autres molécules biologiques d'intérêt est également valable.
Dans le cas des oligonucléotides, le greffage de ces molécules et la mise en évidence de leur présence sur la surface du support solide sont composés de quatre étapes :

(i) l'insolation : c'est l'étape qui permet de libérer localement la fonction réactive B sur la surface du support solide sous l'action de la lumière. Cette fonction B va permettre le greffage de la biomolécule.
(ii) l'immobilisation : c'est l'étape de greffage proprement dite de l'ON par l'intermédiaire de la réaction entre la fonction aldéhyde portée par l'ON et la fonction B photo-déprotégée à la surface du support solide après l'étape d'insolation
(iii) l'hybridation : c'est l'étape de mise en évidence du greffage ou non de l'ON désiré par l'intermédiaire de la reconnaissance de l'ON greffé sur la surface du support solide par son complémentaire portant un fluorophore.
(iv) la détection de la fluorescence au scanner : c'est l'étape du traitement de tout signal émis par le fluorophore.

**I) Mode opératoire :**

1) Etape d'insolation

Le capillaire fonctionnalisé par le composé (11) et tel que préparé ci-dessus à l'exemple 5 a été rempli d'un mélange 1/1 de pyridine/eau.
On a ensuite procédé à une insolation du capillaire à différents endroits choisis de celui-ci durant 5 secondes, à l'aide d'un insolateur à rayonnements ultraviolet A (UVA) à une longueur d'onde de 365 nm, en ajustant la taille de la fente à 160 μm, avec une intensité de 4 mW/mm$^2$ ledit insolateur étant équipé d'une lampe de 100 W.
Le capillaire a ensuite été rincé à l'eau puis séché à l'azote.

2) Etape d'immobilisation des ON

Le capillaire ainsi traité selon la première étape a été incubé avec une solution d'ON portant une fonction aldéhyde à 10 μM dans de l'eau et on a laissé agir pendant 30 minutes.
Le capillaire a ensuite été rincé à l'eau, puis avec une solution de laurylsulfate de sodium à 0,2 %, puis de nouveau à l'eau et enfin séché à l'azote.

1) Première étape : Préparation d'un composé **(12) :** Introduction de l'oxyamine

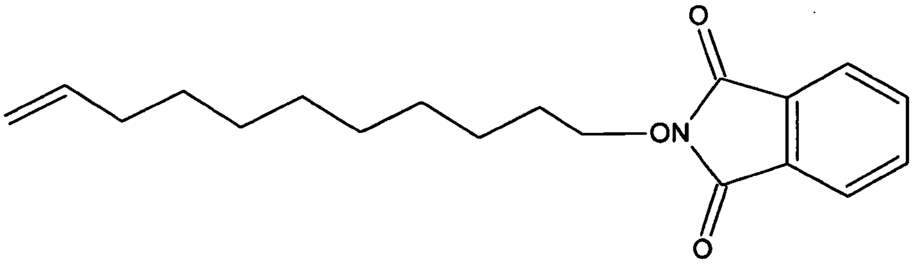

**(12)**

Une solution de *N*-hydroxyphtalimide (3,50 g ; 21 mmoles) et de carbonate de potassium (8 g ; 41 mmoles) dans 250 ml de diméthylformamide (DMF) a été chauffée à une température de 50°C sous argon pendant une heure sous agitation. On a ensuite ajouté du 11-bromo-undécène (5,0 g ; 21 mmoles), puis le mélange réactionnel a de nouveau été agité pendant 3 heures à une température de 50°C. Après filtration, le solvant a été évaporé sous pression réduite. On a ensuite ajouté au résidu ainsi obtenu 100 ml de dichlorométhane puis la phase organique a été lavée avec de la soude 0,1 N, puis avec une solution aqueuse saturée en chlorure de sodium. La phase organique a ensuite été séchée sur sulfate de sodium anhydre puis évaporée. Le produit **(12)** a été obtenu sous la forme d'une poudre blanche (6,45 g ; 20,4 mmoles ; rendement 97 %) présentant un point de fusion compris entre 38 et 40°C.

L'analyse en résonance magnétique nucléaire du proton **(RMN -$^1$H)**, effectuée à une longueur d'onde de 200 MHz dans du CDCL3 était la suivante :

$\delta$ ppm = 1,20-1,50 (12 H, m, 6 $C\underline{H_2}$) ; 1,78 (2 H, m, J = 7 Hz, $C_2\underline{H_3}CH_2C\underline{H_2}CH_2$) ; 2,02 (2 H, m, $C_2\underline{H_3}C\underline{H_2}CH_2$) ; 4,18 (2 H, t, J = 6 Hz, $C\underline{H_2}ON$) ; 4,95 (2 H, m, $C\underline{H_2}CHCH_2$); 5,79 (1 H, m, $CH_2C\underline{H}CH_2$); 7,70-7,85 (4 H, m, Ar-$\underline{H}$).

**SM** (ESI, mode positif) : $M_{calc}$ - 315 ($C_{19}H_{25}NO_3$), m/z = 338 $[M+Na]^+$.

2) <u>Deuxième étape : Hydrazinolyse du composé (12) pour conduire au composé **(13)**</u>

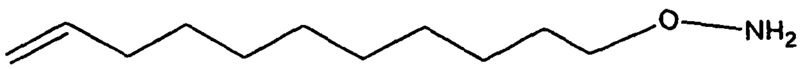

**(13)**

Le composé **(12)** (3,0 g ; 9,64 mmoles) obtenu ci-dessus à la première étape a été solubilisé dans 30 ml de dichlorométhane et l'on a ajouté 966 mg d'hydrazine (19,3 mmoles). La solution obtenue a été portée au reflux pendant 3 heures, puis filtrée et évaporée sous pression réduite. Le produit brut a été purifié par chromatographie sur gel de silice, avec comme éluant un mélange d'acétate d'éthyle et de cyclohexane (v/v : 1/2). On a obtenu le composé (13) sous la forme d'une huile translucide (1,65 g ; 8,94 mmoles) avec un rendement de 93 %.

**RMN-$^1$H** (200 MHz, $CDCl_3$) : $\delta$ ppm = 1,20-1,50 (12 H, m, 4 $C\underline{H_2}$), 1,56 (2 H, m, $C_2\underline{H_3}CH_2C\underline{H_2}CH_2$), 2,03 (2 H, q, $C_2\underline{H_3}C\underline{H_2}CH_2$), 3,65 (2 H, t, J = 6 Hz, $C\underline{H_2}ON$), 4,96 (2 H, m, $C\underline{H_2}CHCH_2$), 5,80 (1 H, m, $CH_2C\underline{H}CH_2$).

SM (ESI, mode positif) : $M_{calc}$ = 185 ($C_{11}H_{23}NO$), m/z = 186 [M+H]+

3) <u>Troisième étape : Protection de l'oxyamine du composé **(13)** pour obtenir le composé **(14)**</u>

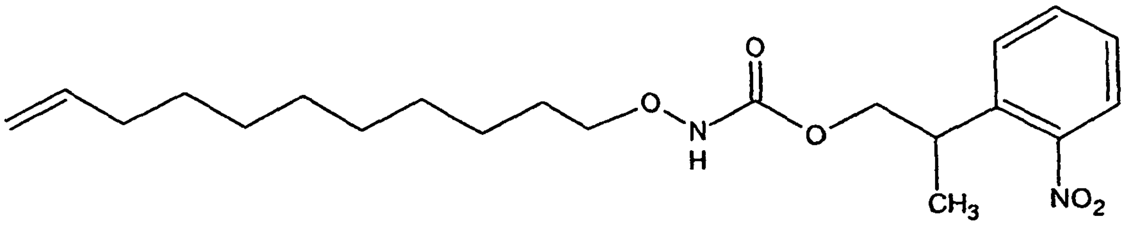

**(14)**

Le composé **(13)** préparé ci-dessus à la deuxième étape (470 mg ; 2,54 mmoles) a été dissous dans 2 ml de pyridine. On a ensuite ajouté, goutte à goutte, 3 ml d'une solution de dichlorométhane contenant 620 mg (5,08 mmoles) de chloroformiate de 2-(2-nitrophényl)-propyle (Cl-NPPOC). Le mélange réactionnel a été agité pendant 2 heures à l'obscurité puis filtré et évaporé sous pression réduite. Le résidu obtenu a ensuite été solubilisé dans du dichlorométhane puis la phase organique a été lavée avec une solution aqueuse de carbonate de sodium à 10 % (p/v), puis avec une solution aqueuse d'acide chlorhydrique (IN), et enfin avec une solution aqueuse saturée en chlorure de sodium. La phase organique a été séchée sur sulfate de sodium anhydre puis évaporée. Le produit brut a été purifié par chromatographie sur gel de silice en utilisant un mélange d'acétate d'éthyle et de cyclohexane (v/v : 1/4) comme éluant. Le produit **(14)** a été obtenu sous la forme d'une huile orange (873 mg ; 2,22 mmoles) avec un rendement de 88 %.

**RMN-$^1$H** (200 MHz, $CDCl_3$) : δ ppm = 1,20-1,40 (12 H, m, 6 $C\underline{H}_2$), 1,35 (3 H, d, J = 7 Hz, $C\underline{H}_3$), 1,56 (2 H, m $C_3H_3CH_2C\underline{H}_2CH_2$), 2,03 (2 H, m, $C_2H_3C\underline{H}_2CH_2$), 3,65 (2 H, m, $C\underline{H}_2ON$, 1H, m, $C\underline{H}$), 4,28 (2H, m, $C\underline{H}_2OCO$), 4,96 (2 H, m, $C\underline{H}_2CHCH_2$), 5,80 (1 H, m, $CH_2C\underline{H}CH_2$), 7,20-7,80 (4 H, m, Ar-$\underline{H}$ NPPOC).

**SM** (ESI, mode positif) : $M_{calc}$ = 392 ($C_{21}H_{32}N_2O_5$), m/z = 415 $[M+Na]^+$, m/z = 410 $[M+NH_4]^+$,

4) Quatrième étape : Hydrosilylation du composé **(14)** pour obtenir le composé **(15)**

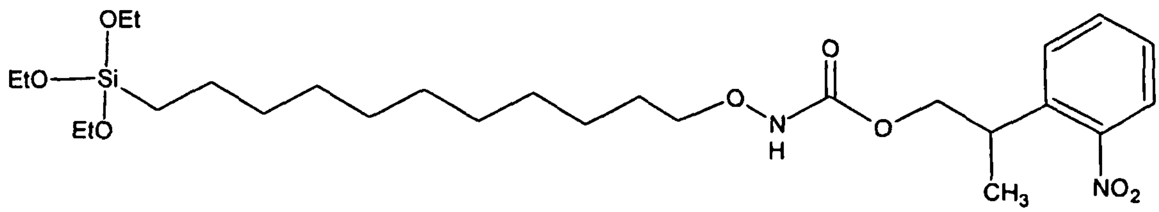

**(15)**

1,28 g (3,2 mmoles) de composé **(14)** obtenu selon le procédé décrit ci-dessus à la troisième étape ont été ajoutés à une solution constituée de 5 ml de triéthoxysilane et de 14 µl (0,25 équivalent) de catalyseur de Karsted. Le mélange réactionnel a été agité pendant 2 heures à une température de 60°C puis le solvant a été évaporé et co-évaporé avec du DMF (5 à 6 fois) sous vide. On a obtenu le produit (15) attendu sous la forme d'une huile (1,49 g ; 2,68 mmoles) avec un rendement de 83 %.

**RMN-$^1$H** (200 MHz, $CDCl_3$) : δ ppm = 0,6 (2 H, m, $C\underline{H}_2Si$), 1,20-1,40 (16 H, m, 6 $C\underline{H}_2$, 9H 3 $CH_3CH_2OSi$), 1,35 (3 H, d, J = 7 Hz, $C\underline{H}_3$), 3,65 (2 H, m, $C\underline{H}_2ON$, 1H, m, $C\underline{H}$), 4,28 (2 H, m, $C\underline{H}_2OCO$), 7,20-7,80 (4 H, m, Ar-$\underline{H}$ NPPOC).

**SM** (ESI, mode positif) : $M_{calc}$ = 556 ($C_{27}H_{48}N_2O_8Si$), m/z = 579 $[M+Na]^+$.

**EXEMPLE 9 : PREPARATION D'UN SUPPORT SOLIDE PLAN FONCTIONNALISE AVEC UN AGENT DE COUPLAGE DE FORMULE (I) COMPORTANT UNE FONCTION D'ACCROCHAGE A DE TYPE SILANE**

Dans cet exemple le composé **(15)** tel que préparé ci-dessus à l'exemple 8 a été utilisé à titre d'agent de couplage de formule (I).

1) Première étape : Réhydratation de la surface (activation)

Un support plan solide en silicium comportant des fonctions inactives $SiO_2$ a été plongé dans une solution constituée de 4 ml d'eau et de 3 ml d'éthanol et renfermant de la soude (1 g NaOH) pendant 1 heure. Le support a

ensuite été rincé à l'eau ultra pure, puis avec une solution d'acide chlorhydrique 0,2 N et enfin à l'eau ce qui a conduit à une surface comportant des fonctions activées SiOH. Le support a ensuite été séché à l'argon.

2) Deuxième étape : silanisation de support avec le composé **(15)**

Le support ainsi activé a été trempé dans une solution du composé **(15)** (7 ml) a 5mM dans un mélange toluène/triéthylamine (v/v : 97/3) pendant une nuit à 80°C. Le support a ensuite été lavé au toluène puis à l'éthanol et enfin séché à l'argon. Le support ainsi fonctionnalisé par le composé (15) a ensuite été mis à l'étuve à 110°C pendant 3 heures.

**EXEMPLE 10 : UTILISATION D'UN SUPPORT PLAN SOLIDE FONCTIONNALISE PAR UN COMPOSE DE FORMULE (I) POUR L'IMMOBILISATION DE MOLECULES BIOLOGIQUES D'INTERET**

Tout comme décrit ci-dessus à l'"exemple 7, le greffage d'ON et la mise en évidence de leur présence à la surface du support solide sont composés de quatre étapes : l'insolation, l'immobilisation, l'hybridation et enfin la détection de la fluorescence au scanner.

**I) Mode opératoire :**

1) Première étape : insolation du support

Le support plan solide, fonctionnalisé par le composé (15) selon le procédé décrit ci-dessus à l'exemple 9, sur lequel a été plaqué un masque en plastique qui comporte des points transparents de taille 100 μm sur 100 μm qui laissent passer la lumière, a été recouvert d'une goutte d'un mélange 1/1 (v/v) de pyridine/eau. On a ensuite procédé à une insolation sur toute la surface du support et donc à travers le masque durant 15 secondes, à l'aide d'un insolateur à rayonnement ultraviolet A (UVA) à une longueur d'onde de 365 nm, avec une intensité de 4 $mW/mm^2$, ledit insolateur étant équipé d'une lampe de 100 W.

Le support plan solide a ensuite été rincé à l'eau puis séché à l'azote.

1) Deuxième étape : Immobilisation des ON

Le support plan ainsi traité selon la première étape a été incubé avec une solution d'ON portant une fonction aldéhyde à 10 μM dans de l'eau et on a laissé agir pendant 30 minutes.

Le support a ensuite été rincé à l'eau, puis avec une solution de laurylsulfate de sodium à 0,2 %, puis de nouveau à l'eau et enfin séché à l'azote.

2) Troisième étape : Hybridation

Le support solide sur lequel ont été immobilisés les ON conformément à l'étape 2) ci-dessus a été rempli avec une solution d'ON complémentaires portant le fluorophore CY3 et on a laissé agir pendant 1 heure à une température de 40°C. Le support a ensuite été rincé avec une solution de citrate de sodium à 0,2 %.

3) Quatrième étape : Lecture

Le support sur lequel les ON marqués au fluorophore se sont hybridés conformément à l'étape 3) ci-dessus a été placé sur une lame de verre puis introduit dans un lecteur de fluorescence vendu par la société Genomic's Solution.

La même mesure a également été réalisée sur un support plan solide témoin (type b/) ayant subi les mêmes traitements que ceux décrits ci-dessus pour le capillaire test (type b/) sauf l'étape de fonctionnalisation par le composé **(15).**

**II) Résultats :**

L'image de la fluorescence obtenue est représentée sur la figure 2 annexée sur laquelle on peut constater que seuls les endroits ayant été insolés sont fluorescents. On voit par ailleurs que seul le support plan solide fonctionnalisé par les agents de couplage (15) conformes à l'Invention (support de type a/) comporte des points fluorescents correspondant à l'immobilisation photo-localisée des ON désirés, le support non fonctionnalisé (support de type b/) étant totalement non fluorescent.

Cet exemple démontre que les supports solides conformes à l'Invention peuvent être avantageusement utilisés

pour l'immobilisation de molécules d'intérêt de façon spécifique et localisée.

## Revendications

1. Agent de couplage de formule (I) suivante :

A-X-B-Proc (I)

dans laquelle :

- A représente une fonction d'accrochage sur un support solide, ladite fonction étant choisie parmi les fonctions de type amine, phosphoramidite, silane et ester activé ;
- X représente un bras espaceur ;
- B est une fonction réactive choisie parmi les groupements conduisant, après photo-déprotection, à une fonction de type oxyamine ($-ONH_2$) et dérivés ou hydrazide ($-NH-NH_2$) et dérivés, ladite fonction B étant protégée par un groupement Proc ;
- Proc est un groupement protecteur photolabile de formule (II) suivante :

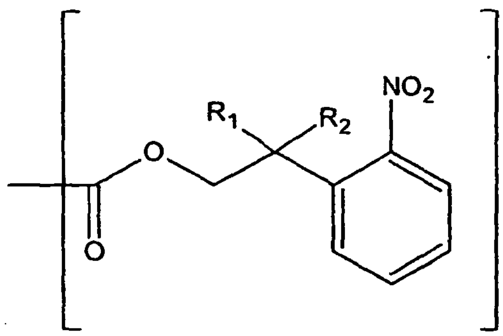

(II)

dans laquelle :

- $R_1$ et $R_2$, identiques ou différents, représentent un atome d'hydrogène ou un radical alkyle en $C_1$-$C_4$.

2. Agent de couplage selon la revendication 1, **caractérisé par le fait que** le bras espaceur X est une chaîne hydrophobe non chargée ou une chaîne hydrophile non chargée.

3. Agent de couplage selon la revendication 2, **caractérisé par le fait que** le bras espaceur X est choisi parmi les chaînes hydrocarbonées saturées comportant de 1 à 13 atomes de carbone et les éthers de glycol dans lesquels la chaîne carbonée comporte de 4 à 12 atomes de carbone.

4. Agent de couplage selon l'une quelconque des revendications précédentes, **caractérisé par le fait que** $R_1$ et $R_2$, indépendamment l'un de l'autre, représentent un radical alkyle choisi parmi les radicaux méthyle et éthyle.

5. Agent de couplage selon l'une quelconque des revendications précédentes, **caractérisé par le fait qu'**il est choisi parmi ceux dans lesquels :

i) - A représente une fonction phosphoramidite,

- X représente une chaîne hydrocarbonée saturée ayant 6 atomes de carbone,
- B représente une fonction oxyamine (-ONH-), et
- $R_1$ et $R_2$ représente un atome d'hydrogène ou un radical méthyle ;

ii) - A représente une fonction trialcoxy($C_1$-$C_4$)silane,

- X représente une chaîne hydrocarbonée saturée ayant de 2 à 12 atomes de carbone,

- B représente une fonction oxyamine (-ONH-), et
- $R_1$ et $R_2$ représente un atome d'hydrogène ou un radical méthyle ;

iii)- A représente une fonction carboxylique estérifiée par du N-hydroxysuccinimide ou du pentafluorobenzène,

- X représente $-CH_2-$,
- B représente une fonction oxyamine (-ONH-), et
- $R_1$ et $R_2$ représente un atome d'hydrogène ou un radical méthyle.

6. Agent de couplage selon la revendication 5, **caractérisés par le fait que** les composés iii) sont choisis parmi les composés dans lesquels la fonction A est du triéthoxysilane et X est une chaîne hydrocarbonée saturée ayant 3 ou 11 atomes de carbone.

7. Utilisation d'au moins un agent de couplage de formule (I) tel que défini à l'une quelconque des revendications 1 à 6, pour la fonctionnalisation de supports solides.

8. Procédé de préparation d'un support solide fonctionnalisé **caractérisé par le fait qu'**il comporte au moins une étape de mise en contact d'au moins une surface d'un support solide avec une solution, dans un solvant organique, d'au moins un agent de couplage de formule (I) tel que défini à l'une quelconque des revendications 1 à 6.

9. Procédé selon la revendication 8, **caractérisé par le fait que** la mise en contact du support solide avec la solution d'agent de couplage de formule (I) est réalisée à une température comprise entre 4 et 80°C pendant 1 à 48 heures.

10. Procédé selon la revendication 8 ou 9, **caractérisé par le fait que** le support solide est choisi parmi les supports de verre, de céramiques, de silicium ou de plastique, lesdits supports comportant au moins une surface hydratée, hydroxylée, silanisée, aminée ou de type ester activé.

11. Procédé selon l'une quelconque des revendications 8 à 10, **caractérisé par le fait que** le support possède au moins une surface plane ou non, lisse ou structurée, et se présente sous la forme de lame de verre, de plaque plastique plane ou à puits, de capillaire ou de bille poreuse ou non.

12. Support solide **caractérisé par le fait qu'**il comporte au moins une surface fonctionnalisée par un ou plusieurs agents de couplage de formule (I) tels que définis à l'une quelconque des revendications 1 à 6.

13. Utilisation d'au moins un support solide tel que défini à la revendication 12 pour l'immobilisation covalente de molécules biologiques d'intérêt comportant une fonction chimique complémentaire vis-à-vis de la fonction réactive B des agents de couplage de formule (I) présents à la surface dudit support solide.

14. Utilisation selon la revendication 13, **caractérisée par le fait que** les molécules biologiques d'intérêt sont des molécules d'acide nucléique.

15. Procédé d'immobilisation de molécules biologiques d'intérêt sur un support solide tel que défini à la revendication 14, **caractérisé en ce qu'**il comprend au moins une première étape de photo-déprotection des fonctions réactives B composés de formule (I) par insolation d'au moins une partie de la surface du support solide, puis la mise en contact du support solide ainsi activé avec une solution de molécules biologiques d'intérêt, pour conduire à l'immobilisation desdites molécules par formation d'une liaison covalente entre au moins une fonction chimique portée par lesdites molécules et réactive vis-à-vis des fonctions réactives B desdits composés de formule (I), sur ledit support solide et la répétition éventuelle de ces deux étapes.

16. Procédé selon la revendication 15, **caractérisé par le fait que** l'étape de photo-déprotection est localisée sur seulement une partie de la surface fonctionnalisée du support solide.

17. Procédé selon la revendication 15 ou 16, **caractérisé par le fait que** l'étape d'immobilisation est réalisée par la formation de liaisons oxime entre les fonctions B oxyamine des composés de formule (I) et les fonctions carbonylées des molécules biologiques d'intérêt.

18. Procédé selon la revendication 17, **caractérisé par le fait que** l'étape d'immobilisation est réalisée à un pH compris entre 4 et 7.

**19.** Procédé selon l'une quelconque des revendications 16 à 18, **caractérisé par le fait que** la longueur d'onde d'insolation est comprise entre 300 et 400 nm.

**20.** Support solide **caractérisé par le fait qu'**il est obtenu selon le précédé tel que défini à l'une quelconque des revendications 15 à 19 et **par le fait qu'**il comporte au moins une surface sur laquelle des molécules biologiques d'intérêt sont immobilisées par l'intermédiaire d'une liaison covalente formée avec la fonction réactive B des agents de couplage de formule (I) tels que définis à l'une quelconque des revendications 1 à 6.

## Claims

**1.** Coupling agent of the following formula (I):

A-X-B-Proc (I)

in which:

- A represents a function for attachment to a solid support, said function being chosen from functions of amine, phosphoramidite, silane and activated ester type;
- X represents a spacer arm;
- B is a reactive function chosen from groups leading, after photo-deprotection, to a function of the type oxyamine ($-ONH_2$) and derivatives or hydrazide ($-NH-NH_2$) and derivatives, said function B being protected by a Proc group;
- Proc is a photolabile protecting group of the following formula (II):

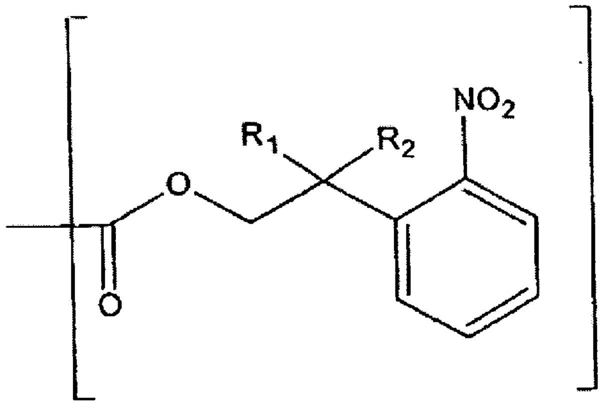

(II)

in which:

- $R_1$ and $R_2$, which may be identical or different, represent a hydrogen atom or a $C_1$-$C_4$ alkyl radical.

**2.** Coupling agent according to claim 1, **characterised in that** the spacer arm X is an uncharged hydrophobic chain or an uncharged hydrophilic chain.

**3.** Coupling agent according to claim 2, **characterised in that** the spacer arm X is chosen from saturated hydrocarbon-based chains comprising 1 to 13 carbon atoms, and glycol ethers in which the carbon-based chain comprises 4 to 12 carbon atoms.

**4.** Coupling agent according to any one of the preceding claims, **characterised in that** $R_1$ and $R_2$, independently of each other, represent an alkyl radical chosen from methyl and ethyl radicals.

**5.** Coupling agent according to any one of the preceding claims, **characterised in that** it is chosen from those in which:

i)

- A represents a phosphoramidite function,
- X represents a saturated hydrocarbon-based chain having 6 carbon atoms,
- B represents an oxyamine function (-ONH-), and
- $R_1$ and $R_2$ represent a hydrogen atom or a methyl radical;

ii)

- A represents a tri($C_1$-$C_4$)alkoxysilane,
- X represents a saturated hydrocarbon-based chain having 2 to 12 carbon atoms,
- B represents an oxyamine function (-ONH-), and
- $R_1$ and $R_2$ represent a hydrogen atom or a methyl radical;

iii)

- A represents a carboxylic function esterified by N-hydroxysuccinimide or pentafluorobenzene,
- X represents $-CH_2-$,
- B represents an oxyamine function (-ONH-), and
- $R_1$ and $R_2$ represent a hydrogen atom or a methyl radical.

**6.** Coupling agent according to claim 5, **characterised in that** the compounds iii) are chosen from compounds in which the function A is triethoxysilane and X is a saturated hydrocarbon-based chain having 3 or 11 carbon atoms.

**7.** Use of at least one coupling agent of the formula (I) as defined in any one of the claims 1 to 6 for the functionalisation of solid supports.

**8.** Process for preparing a functionalised solid support, **characterised in that** it comprises at least one step of bringing at least one surface of a solid support into contact with a solution, in an organic solvent, of at least one coupling agent of formula (I) as defined in any one of the claims 1 to 6.

**9.** Process according to claim 8, **characterised in that** the bringing into contact of the solid support with the solution of coupling agent of formula (I) is carried out at a temperature of between 4 and 80°C for 1 to 48 hours.

**10.** Process according to claim 8 or 9, **characterised in that** the solid support is chosen from supports made of glass, ceramics, silicon or plastic, said supports comprising at least one hydrated, hydroxylated, silanised or aminated surface or a surface of activated ester type.

**11.** Process according to one of the claims 8 to 10, **characterised in that** the support has at least one planar or non-planar, smooth or structured surface, and is in the form of a glass slide, a planar plastic plate or a plastic plate with wells, a capillary tube or a porous or non-porous bead.

**12.** Solid support **characterised in that** it comprises at least one surface functionalised with one or more coupling agents of formula (I) as defined in any one of the claims 1 to 6.

**13.** Use of at least one solid support as defined in claim 12 for covalent immobilisation of biological molecules of interest comprising a chemical function which is complementary in relation to the reactive function B of the coupling agents of formula (I) present at the surface of said solid support.

**14.** Use according to claim 13, **characterised in that** the biological molecules of interest are nucleic acid molecules.

**15.** Process for immobilising biological molecules of interest on a solid support as defined in claim 14, **characterised in that** it comprises at least a first step of photo-deprotection of the reactive functions B of the compounds of formula I) by exposure of at least a part of the surface of the solid support, followed by bringing the thus activated solid support into contact with a solution of biological molecules of interest, in order to lead to the immobilisation of said molecules through the formation of a covalent bond between at least one chemical function that is carried by said molecules and is reactive in relation to the reactive functions B of said compounds of formula (I), on said solid support, and the possible repetition of these two steps.

**16.** Process according to claim 15, **characterised in that** the photo-deprotection step is localised on only a part of the

functionalised surface of the solid support.

**17.** Process according to claim 15 or 16, **characterised in that** the immobilisation step is realised by the formation of oxime bonds between the oxyamine functions B of the compounds of formula (I) and the carbonylated functions of the biological molecules of interest.

**18.** Process according to claim 17, **characterised in that** the immobilisation step is realised at a pH between 4 and 7.

**19.** Process according to any one of the claims 16 to 18, **characterised in that** the exposure wavelength is between 300 and 400 nm.

**20.** Solid support **characterised in that** it is obtained according to the process defined in any one of the claims 15 to 19 and **in that** it comprises at least one surface on which biological molecules of interest are immobilised by means of a covalent bond formed with the reactive function B of the coupling agents of formula (I) as defined in any one of the claims 1 to 6.

**Patentansprüche**

**1.** Kopplungsmittel der folgenden Formel (I):

A-X-B-Proc (1)

in welcher:

- A eine Kupplungsfunktion auf einem festen Trägermaterial wiedergibt, wobei die Funktion ausgewählt ist aus Funktionen vom Typ Amin, Phosphoramidit, Silan und aktiviertem Ester;
- X einen Spacer wiedergibt;
- B eine reaktive Funktion ist, ausgewählt aus leitenden Gruppierungen, nach Photoentschützung, einer Funktion vom Typ Oxyamin (-$ONH_2$) und Derivaten, oder Hydrazid (-NH-$NH_2$) und Derivaten, wobei die Funktion B durch eine Proc-Gruppierung geschützt ist;
- Proc eine photolabile Schutzgruppe der folgenden Formel (II) ist:

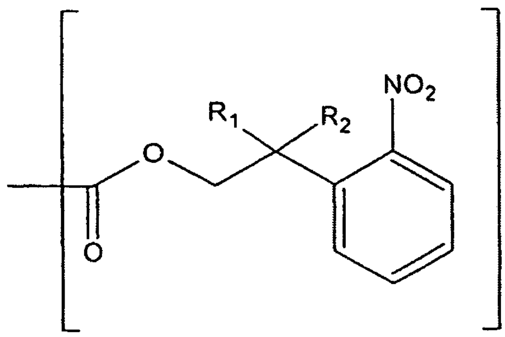

(II)

in welcher
- $R_1$ und $R_2$ gleich oder verschieden ein Wasserstoffatom oder ein $C_1$ - $C_4$- Alkylradikal wiedergeben.

**2.** Kopplungsmittel nach Anspruch 1, **dadurch gekennzeichnet, dass** der Spacer X eine hydrophobe, nicht geladene Kette oder eine hydrophile, nicht geladene Kette ist.

**3.** Kopplungsmittel nach Anspruch 2, **dadurch gekennzeichnet, dass** der Spacer X ausgewählt ist aus gesättigten Kohlenwasserstoffketten, die 1 bis 13 Kohlenstoffatome enthalten, und Glycolethern, in denen die Kohlenstoffkette 4 bis 12 Kohlenstoffatome enthält.

**4.** Kopplungsmittel nach einem der vorhergehenden Ansprüche, **dadurch gekennzeichnet, dass** $R_1$ und $R_2$ unab-

hängig voneinander ein Alkylradikal wiedergeben, ausgewählt aus Methyl- und Ethylradikalen.

5. Kopplungsmittel nach einem der vorhergehenden Ansprüche, **dadurch gekennzeichnet, dass** es aus solchen ausgewählt ist, bei denen:

i) A eine Phosphoramidit-Funktion wiedergibt,

- X eine gesättigte Kohlenwasserstoffkette mit 6 Kohlenstoffatomen wieder gibt,
- B eine Oxyaminfunktion (-ONH-) wiedergibt, und
- $R_1$ und $R_2$ ein Wasserstoffatom oder ein Methylradikal wiedergeben;

ii) - A eine Trialkoxy($C_1$-$C_4$)silan-Funktion wiedergibt,

- X eine gesättigte Kohlenwasserstoffkette mit 2 bis 12 Kohlenstoffatomen wiedergibt,
- B eine eine Oxyaminfunktion (-ONH-) wiedergibt, und
- $R_1$ und $R_2$ ein Wasserstoffatom oder ein Methylradikal wiedergeben;

iii) - A eine mit N-Hydroxysuccinimid oder Pentafluorbenzol veresterte Carboxylfunktion wiedergibt,

- X $CH_2$ wiedergibt,
- B eine Oxyaminfunktion (-ONH-) wiedergibt, und
- $R_1$ und $R_2$ ein Wasserstoffatom oder ein Methylradikal wiedergeben.

6. Kopplungsmittel nach Anspruch 5, **dadurch gekennzeichnet, dass** die Verbindungen iii) ausgewählt sind aus Verbindungen, in denen die Funktion A Triethoxysilan ist und X eine gesättigte Kohlenwasserstoffkette mit 3 oder 11 Kohlenstoffatomen ist.

7. Verwendung wenigstens eines Kopplungsmittels der Formel (I) gemäß einem der Ansprüche 1 bis 6 für die Funktionalisierung von festen Trägermaterialien.

8. Herstellungsverfahren für ein funktionalisiertes festes Trägermaterial, **dadurch gekennzeichnet, dass** es wenigstens einen Schritt enthält, in dem wenigstens eine Oberfläche des festen Trägermaterials mit einer Lösung wenigstens eines Knpplungsmittels der Formel (I) gemäß den Ansprüchen 1 bis 6, in einem organischen Lösungsmittel, in Kontakt gebracht wird.

9. Verfahren nach Anspruch 8, **dadurch gekennzeichnet, dass** das in Kontakt bringen des festen Trägermaterials mit der Lösung des Kopplungsmittels der Formel (I) bei einer Temperatur zwischen 4 und 80°C während 1 bis 48 Stunden durchgeführt wird.

10. Verfahren nach Anspruch 8 oder 9, **dadurch gekennzeichnet, dass** das feste Trägermaterial ausgewählt ist aus Trägermaterialien aus Glas, Keramik, Silicium oder Kunststoff, wobei die Trägermaterialien wenigstens eine hydratisierte, hydroxylierte, silanisierte, aminierte Oberfläche aufweisen, oder eine vom Typ des aktivieren Esters.

11. Verfahren nach einem der Ansprüche 8 bis 10, **dadurch gekennzeichnet, dass** das Trägermaterial wenigstens eine Oberfläche aufweist, die eben ist oder nicht, glatt oder strukturiert, und die in Form einer Glasklinge, einer ebenen Kunststoffplatte oder einer mit Senken, eines Kapillargefäßes oder einer porösen oder nicht porösen Kugel vorliegt.

12. Festes Trägermaterial, **dadurch gekennzeichnet, dass** es wenigstens eine, durch ein oder mehrere Kopplungsmittel der Formel (I) gemäß einem der Ansprüche 1 bis 6, funktionalisierte Oberfläche enthält.

13. Verwendung wenigstens eines festen Trägermaterials gemäß Anspruch 12 für die kovalente Immobilisierung von biologischen Molekülen von Interesse, die eine chemische Funktion enthalten, die komplementär zu der reaktiven Funktion B der Kopplungsmittel der Formel (I) ist, die auf der Oberfläche des festen Trägermaterials vorliegt.

14. Verwendung nach Anspruch 13, **dadurch gekennzeichnet, dass** die biologischen Moleküle von Interesse Nukleinsäuremoleküle sind.

**15.** Verfahren der Immobilisierung von biologischen Molekülen von Interesse auf einem festen Trägermaterial gemäß Anspruch 14, **dadurch gekennzeichnet, dass** es wenigstens einen ersten Schritt der Photoentschützung der reaktiven Funktionen B der Verbindungen der Formel (I) durch Belichten wenigstens eines Teils der Oberfläche des festen Trägermaterials umfasst, dann in Kontakt bringen des auf diese Weise aktivierten festen Trägermaterials mit einer Lösung der biologischen Moleküle von Interesse, um die Immobilisierung der Moleküle durch Bildung einer kovalenten Bindung zwischen wenigstens der von den Molekülen getragenen chemischen Funktion und die gegenüber den reaktiven Funktionen B der Verbindungen der Formel (I) reaktiv ist, auf dem festen Trägermaterial durchzuführen, und das eventuelle Wiederholen der beiden Schritte.

**16.** Verfahren nach Anspruch 15, **dadurch gekennzeichnet**, das der Schritt der Photoentschützung nur an einem Teil der funktionalisieren Oberfläche des festen Trägermaterials durchgeführt wird.

**17.** Verfahren nach Anspruch 15 oder 16, **dadurch gekennzeichnet, dass** der Schritt des Immobilisierens durch die Bildung von Oxim-Bindungen zwischen den Oxyamin-Funktionen B der Verbindungen der Formel (I) und den Carbonylfunktionen der biologischen Moleküle von Interesse realisiert wird.

**18.** Verfahren nach Anspruch 17, **dadurch gekennzeichnet, dass** der Schritt des Immobilisierens bei einem pH zwischen 4 und 7 durchgeführt wird.

**19.** Verfahren nach einem der Ansprüche 16 bis 18, **dadurch gekennzeichnet, dass** die Wellenlänge der Strahlung zwischen 300 und 400 nm liegt.

**20.** Festes Trägermaterial, **dadurch gekennzeichnet, dass** es durch ein Verfahren gemäß den Ansprüchen 15 bis 19 erhalten wird, und **dadurch**, dass es wenigstens eine Oberfläche umfasst, auf der biologische Moleküle von Interesse durch ein Zwischenprodukt einer kovalenten Bindung immobilisiert sind, die mit der reaktiven Funktion B des Kopplungmittels der Formel (I) gemäß einem der Anspruche 1 bis 6 gebildet wird.

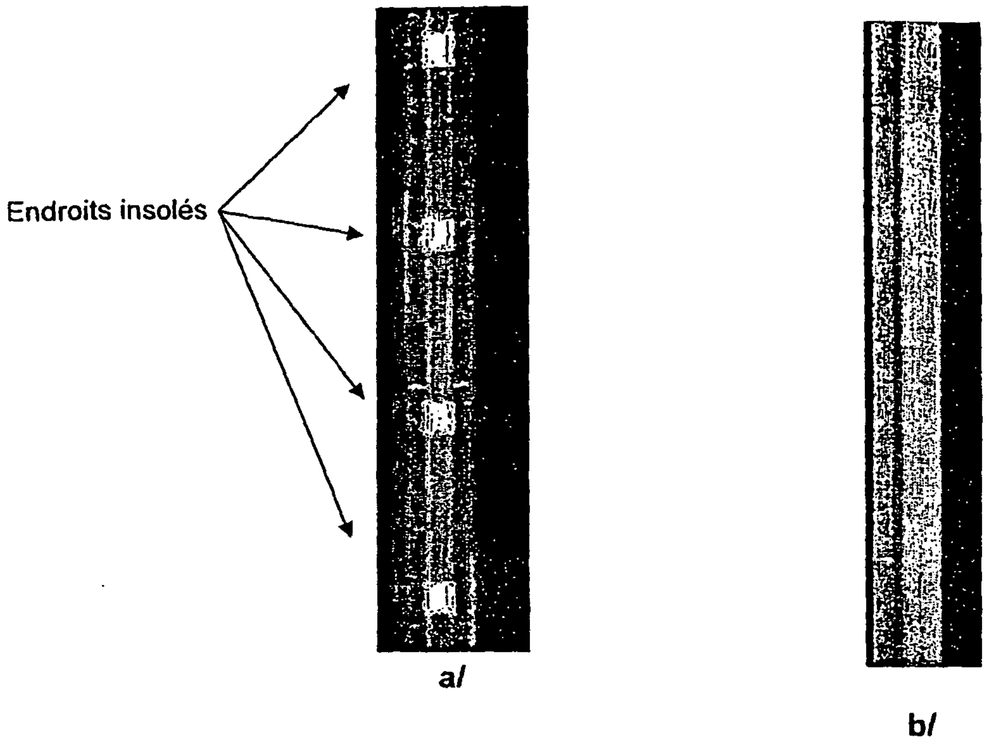


FIGURE 1

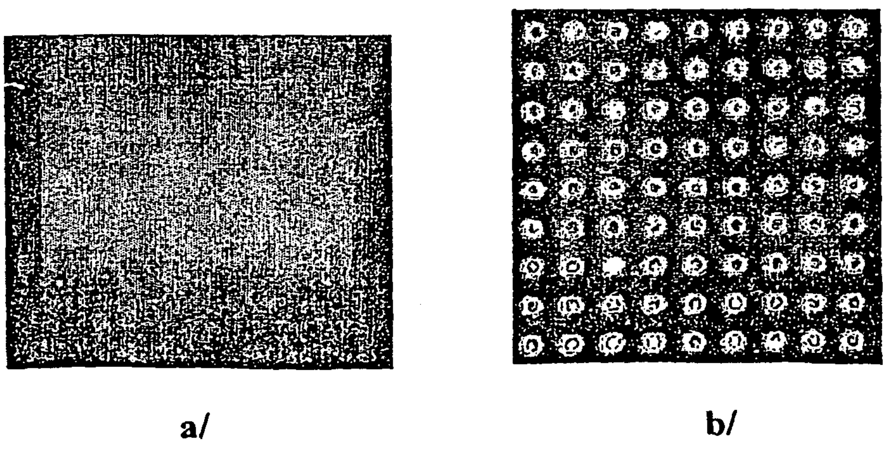


FIGURE 2

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9739151 A
- FR 2703359


### Littérature non-brevet citée dans la description


- **Bhushan K. R. et al.** *Tetrahedon Letters,* 2003, vol. 44, 8585-8588
- **Acedo M. et al.** *Tetrahedon Letters,* 1992, vol. 33 (34), 4989-4992
- **A. ULMAN.** *Chem. Rev.,* 1996, vol. 96, 1533-1554